# EUROPÄISCHE PATENTSCHRIFT

(11) **EP 3 638 768 B1**
(45) Veröffentlichungstag und Bekanntmachung des Hinweises auf die Patenterteilung: **07.04.2021**
(21) Anmeldenummer: 18732309.2
(22) Anmeldetag: 13.06.2018
(51) Int. Cl.: C12M 1/12, C12M 3/06, C12M 1/00, C12M 3/04

(54) **VORRICHTUNG UND VERFAHREN FÜR DIE KULTIVIERUNG VON ZELLEN**
DEVICE AND METHOD FOR CULTIVATING CELLS
DISPOSITIF ET PROCÉDÉ POUR LA CULTURE DE CELLULES

(30) Priorität: 13.06.2017 DE 102017209942
(43) Veröffentlichungstag der Anmeldung: 22.04.2020
(73) Patentinhaber: Fraunhofer-Gesellschaft zur Förderung der angewandten Forschung e.V., 80686 München (DE)
(72) Erfinder: LOSKILL, Peter, 70199 Stuttgart (DE); SCHNEIDER, Oliver, 87435 Kempten (DE); SCHNEIDER, Stefan, 71116 Gärtringen (DE)
(74) Vertreter: Schrell, Andreas
(86) Internationale Anmeldenummer: PCT/EP2018/065718
(87) Internationale Veröffentlichungsnummer: WO 2018/229157

(56) Entgegenhaltungen:
- WO-A1-99/55827
- WO-A2-02/102969
- GB-A- 2 350 678
- US-A1- 2005 106 714
- US-A1- 2010 323 434
- KIM JIN-YOUNG ET AL: "3D spherical microtissues and microfluidic technology for multi-tissue experiments and analysis", JOURNAL OF BIOTECHNOLOGY, ELSEVIER, AMSTERDAM, NL, Bd. 205, 12. Januar 2015 (2015-01-12), Seiten 24-35, XP029240257, ISSN: 0168-1656, DOI: 10.1016/J.JBIOTEC.2015.01.003

## Beschreibung

Die vorliegende Erfindung betrifft eine Vorrichtung zur Kultivierung von Zellen, insbesondere Geweben, umfassend eine Trägerplatteneinheit, die eine zentrale Rotationsachse, mindestens eine proximal zur Rotationsachse angeordnete Zugangsöffnung, mindestens eine distal zur Rotationsachse angeordnete Kultivierungskammer und mindestens einen die Zugangsöffnung mit der Kultivierungskammer verbindenden Kanal aufweist, sowie ein Verfahren zur Kultivierung von Zellen in einer erfindungsgemäßen Vorrichtung und ein Verfahren zur Herstellung der erfindungsgemäßen Vorrichtung.

Mikrofluidische Organ-on-a-chip Systeme, auch TissueChips, oder Mikrophysiologische Systeme (MPS) genannt, in denen Zellen und Gewebe in kleinstem Maßstab kultiviert und pharmazeutische oder kosmetische Substanzen getestet werden können, haben sich in den letzten Jahren von einer konzeptionellen Idee zu einer disruptiven neuen Technologie entwickelt. Ihnen wird ein hohes Potential als Tierversuchsalternative und Screening Tool in der pharmazeutischen Entwicklung, bei der Toxizitätsbestimmung und in der personalisierten Medizin zugeschrieben. Das Grundprinzip von Organ-on-a-chip-Systemen besteht in der Schaffung einer kontrollierten, mikrometer-dimensionierten Umgebung zur Kultivierung von menschlichem und tierischem Organgewebe und Zellen.

Für die Integration beziehungsweise Erzeugung von Geweben in den Organ-on-a-chip Systemen müssen Zellen, Zellhaufen oder Gewebebestandteile in die mikrometer-dimensionierten Umgebungen eingebracht werden. Um physiologische Zelldichten entsprechend dem in vivo Gewebe zu erzeugen müssen, speziell im Fall von nicht proliferierenden Zelltypen, die Zellen, Zellhaufen oder Gewebebestandteile bereits initial in hoher Dichte injiziert werden. Dies ist oft nur durch komplizierte Methoden mit hoher Variabilität und großer Belastung der injizierten Zellen möglich.

In den meisten Fällen, insbesondere bei proliferierenden Zellen, werden die Zellen in variabler Dichte injiziert und die gewünschte Zelldichte daraufhin durch Differenzierung und/oder Proliferation der Zellen im Chip erreicht. Um die Zellen in die Kulturkammern zu transportieren und/oder dort initial hohe Zelldichten zu erzielen, werden im Stand der Technik Druckgradienten erzeugt, sodass Zellsuspensionen mit positivem Druck in die Mikrokanäle injiziert oder mit negativem Druck in die Kanäle hinein gesaugt werden (PCT/US2014/047482; A. Mathur, P. Loskill, K. Shao, N. Huebsch, S. Hong, S. G. Marcus, N. Marks, M. Mandegar, B. R. Conklin, L. P. Lee, et al., Sci. Rep. 2015, 5, 8883). Alternativ können einzelne Zellen auch mithilfe von Bioprinting direkt auf den Chip aufgebracht werden (S. Knowlton, B. Yenilmez, S. Tasoglu, Trends Biotechnol. 2016, 34, 685-688) oder vorgeformte Spheroide injiziert werden (N. S. Bhise, J. Ribas, V. Manoharan, Y. S. Zhang, A. Polini, S. Massa, M. R. Dokmeci, A. Khademhosseini, J. Control. Release 2014, 190, 82-93; J.-Y. Kim, D. A. Fluri, R. Marchan, K. Boonen, S. Mohanty, P. Singh, S. Hammad, B. Landuyt, J. G. Hengstler, J. M. Kelm, et al., J. Biotechnol. 2015, 205, 24-35).

Die Druckschrift GB 2 350 678 offenbart ein Verfahren zur Zellkultivierung und beschreibt eine Mikrofluidik-Vorrichtung. Die Zellkulturkammer befindet sich relativ zur Drehachse nach Außen beabstandet und die Fluidauslässe befinden sich an der Disk-Peripherie. Die Ein- und Auslässe sowie die Kulturkammer sind über radial zueinander verlaufende Kanäle miteinander verbunden und auf einer Kreisbahn zueinander angeordnet.

Die im Stand der Technik bekannten Verfahren und Vorrichtungen zur Kultivierung von Zellen, insbesondere zur Kultivierung und Bereitstellung hoher Zelldichten, insbesondere zur Herstellung von Zellverbänden und Geweben, sind häufig hinsichtlich der eingesetzten, die Lebens- und/oder Kulturfähigkeit der Zellen beeinträchtigenden Zellkulturbedingungen, beispielsweise erhöhten Scherkräften und/oder Druckgradienten, nachteilig.

Das der vorliegenden Erfindung zugrundeliegende technische Problem ist daher die Bereitstellung einer verbesserten Vorrichtung zum Kultivieren von Zellen, die die vorgenannten Nachteile im Stand der Technik überwindet, insbesondere es ermöglicht, Zellen zu kultivieren, ohne diese einer nachteiligen Belastung, zum Beispiel undefiniertem Druck oder Scherkräften auszusetzen.

Insbesondere liegt das zugrundeliegende technische Problem darin eine Vorrichtung bereitzustellen, die in der Lage ist, Zellen in besonders hoher, bevorzugt physiologischer Zelldichte zu kultivieren und vorzugsweise Zellverbände, insbesondere Gewebe, mikroskopische Gewebe, Organe oder Organäquivalente herzustellen. Technisches Problem der vorliegenden Erfindung ist es auch, entsprechende Verfahren zur Kultivierung von Zellen, insbesondere auch zur Herstellung von Zellverbänden, insbesondere Geweben, bereitzustellen. Ein weiteres technisches Problem der vorliegenden Erfindung ist es, Verfahren zur Herstellung der vorgenannten Vorrichtung bereitzustellen.

Die vorliegende Erfindung löst das ihr zugrundeliegende technische Problem durch die Bereitstellung einer Vorrichtung für die Kultivierung von Zellen gemäß Anspruch 1, insbesondere eines, vorzugsweise dreidimensionalen, Zellkultursystems, umfassend eine eine zentrale Rotationsachse aufweisende Trägerplatteneinheit mit mindestens einer proximal zu der Rotationsachse angeordneten Zugangsöffnung, mindestens einer distal zu der Rotationsachse angeordneten Kultivierungskammer und mindestens einem die mindestens eine Zugangsöffnung mit der mindestens einen Kultivicrungskammcr verbindenden Kanal. In besonders bevorzugter Ausführungsform ist die Vorrichtung für die Kultivierung von Zellen eine eine Rotationsachse aufweisende Trägerplatteneinheit, insbesondere Trägerplatte, mit mindestens einer proximal zu der Rotationsachse angeordneten Zugangsöffnung, mindestens einer distal zu der Rotationsachse angeordneten Kultivierungskammer und mindestens einem die mindestens eine Zugangsöffnung mit der mindestens einen Kultivierungskammer verbindenden Kanal. Insbesondere ist die erfindungsgemäße Vorrichtung so ausgebildet, dass sie um ihre zentrale Rotationsachse gedreht werden kann, das heißt in Rotation versetzt werden kann.

Die erfindungsgemäß vorgesehene Vorrichtung für die Kultivierung von Zellen umfasst daher eine eine zentrale Rotationsachse aufweisende Trägerplatteneinheit, insbesondere besteht aus dieser, wobei die Trägerplatteneinheit mindestens eine proximal zu der Rotationsachse angeordnete, von außerhalb der Trägerplatteneinheit zugängliche, Zugangsöffnung, mindestens eine distal zu der Rotationsachse angeordnete Kultivierungskammer und mindestens einen die mindestens eine Zugangsöffnung mit der mindestens einen Kultivierungskammer verbindenden, vorzugsweise radial ausgerichteten, Kanal aufweist. Die mindestens eine Zugangsöffnung, die mindestens eine Kultivierungskammer und der mindestens eine diese beiden Strukturen verbindende Kanal sind in dem Körper der Trägerplatteneinheit, insbesondere der Trägerplatte, oder an ihrer Oberfläche ausgebildet. In bevorzugter Ausführungsform ist vorgesehen, dass die mindestens eine Zugangsöffnung räumlich in der Nähe, das heißt proximal, zu der Rotationsachse angeordnet ist, während die mindestens eine Kultivierungskammer distal, das heißt von der Rotationsachse weiter entfernt angeordnet ist, also in einem peripheren Bereich der Trägerplatteneinheit vorliegt. Der Abstand (r₁) der mindestens einen Zugangsöffnung zu der Rotationsachse ist daher geringer als der Abstand (r₂) der mindestens einen Kultivierungskammer zu der Rotationsachse. Die erfindungsgemäß vorgesehene räumliche Anordnung der mindestens einen Zugangsöffnung zu der mindestens einen Kultivierungskammer führt bei Rotation der Vorrichtung um ihre Rotationsachse dazu, dass aufgrund der Zentrifugalkraft die in die Zugangsöffnung eingebrachten Zellen, Zellkulturmedium, oder Zellsuspension, in Richtung der Fliehkraft in die peripheren Bereiche der Trägerplatteneinheit befördert werden. Darüber hinaus werden störende Luftblasen in entgegengesetzter Richtung, das heißt in Richtung der Zugangsöffnung, aus der Vorrichtung entfernt. Insbesondere gelangen die in die Zugangsöffnung eingebrachten Zellen mithilfe der Zentrifugalkraft über den die mindestens eine Zugangsöffnung mit der mindestens einen Kultivierungskammer verbindenden mindestens einen Kanal in die mindestens eine Kultivicrungskammcr. Sofern, wie in erfindungsgemäß bevorzugter Ausführungsform vorgesehen, mehr als eine Zugangsöffnung und mehr als eine Kultivierungskammer vorgesehen sind, gilt bevorzugt für jede einzelne der Zugangsöffnungen und Kultivierungskammern, dass die Kultivierungskammern in größerer Distanz zur Rotationsachse angeordnet sind, als die Zugangsöffnungen, das heißt dass die Kultivierungskammern distal zur Rotationsachse und die Zugangsöffnungen proximal zu dieser angeordnet sind. Insbesondere befindet sich die mindestens eine Zugangsöffnung im Abstand r₁ von der zentralen Rotationsachse und die mindestens eine Kultivierungskammer in Abstand r₂ von der zentralen Rotationsachse, wobei r₁ < r₂ ist.

Vorteilhafterweise werden die durch die Zugangsöffnung in die Trägerplatteneinheit eingebrachten Zellen, insbesondere die Zellsuspension, gegebenenfalls auch Zellkulturmedium, also während einer Rotation der erfindungsgemäßen Vorrichtung, das heißt durch die Zentrifugalkraft (F_{z} = m ω²r), von der mindestens einen proximal zur Rotationsachse angeordneten Zugangsöffnung durch den mindestens einen die Zugangsöffnung mit der Kultivierungskammer verbindenden Kanal in die mindestens eine distal zur Rotationsachse angeordnete Kultivierungskammer transportiert. Die Rotation mit Winkelgeschwindigkeit ω bewirkt im Abstand r₂ der mindestens einen Kultivierungskammer eine Beschleunigung der Zellen oder Zellsuspension von a = ω² r₂. Vorteilhafterweise akkumulieren sich die Zellen dadurch in der mindestens einen Kultivierungskammer, also im peripheren Bereich der Trägerplatteneinheit. Die Zellen werden vorteilhafterweise durch Zentrifugalkraft in der mindestens einen Kultivierungskammer akkumuliert, ohne dass undefinierte Drücke oder Scherkräfte auf die Zellen einwirken. Die Zellen können dann in der Kultivierungskammer, vorzugsweise mit Versorgung durch Kulturmedium, kultiviert werden, entweder bei geringerer Rotation oder ohne eine weitere Rotation der Vorrichtung. Die Kultivierung der Zellen führt bevorzugt dazu, dass die Zellen die mindestens eine Kultivierungskammer vollständig ausfüllen und so einen dreidimensionalen Zellverband bilden. Vorteilhafterweise ermöglicht die erfindungsgemäße Vorrichtung, reproduzierbar und parallelisierbar Gewebe und Zellverbände mit hoher Zelldichte in der mindestens einen Kultivierungskammer herzustellen, wobei die Vorrichtung zusätzlich einfach bedienbar ist.

Die erfindungsgemäße Vorrichtung ist unter anderem insofern vorteilhaft, als dass sie insbesondere eine Parallelisierung von Zellkulturvorrichtungen, insbesondere Organ-on-a-chip-Systemen, durch gleichzeitiges Einbringen von Zellen in eine Vielzahl von Kultivierungskammern ermöglicht. Die erfindungsgemäße Vorrichtung und Verfahrensweise vereinfacht die Handhabung von mikrophysiologischen in vitro-Zellkultursystemen, insbesondere auch dadurch, dass keine Pumpen für die Injektion, das heißt das Einbringen der Zellen, verwendet werden, Luftblasen durch die Rotation aus der Vorrichtung eliminiert werden können, sowie eine kurze Verfahrensdauer, eine einfachere Handhabbarkeit erreicht und lediglich eine bauliche Einheit verwendet wird. Vorteilhafterweise ergibt sich durch die Rotation der Vorrichtung auch, dass Lufteinschlüsse in den Kultivierungskammern auf einfache Art und Weise entfernt oder vermieden werden können.

Die erfindungsgemäßen Vorrichtungen und Verfahren können insbesondere auch als Tierversuchsalternativen, für Grundlagenstudien aber auch in der angewandten Forschung und Entwicklung verwendet werden, ebenso als Screeningsysteme für therapeutische Präparate oder Chemikalien. In besonders bevorzugter Ausführungsform kann die erfindungsgemäße Vorrichtung und das erfindungsgemäße Verfahren zur Kultivierung von Zellen, insbesondere zur Herstellung von Zellverbänden, zur Erzeugung von künstlichen Geweben oder von Mischgeweben, insbesondere auch für die regenerative und/oder personalisierte Medizin Verwendung finden.

Im Zusammenhang mit der vorliegenden Erfindung wird unter dem Begriff der zentralen Rotationsachse diejenige Gerade verstanden, um die sich die Vorrichtung drehen kann und die insbesondere der Symmetrieachse der makroskopischen Form der Vorrichtung mit dem maximalen Hauptträgheitsmoment entspricht und die durch den geometrischen Mittelpunkt der Vorrichtung verläuft. Insbesondere steht die zentrale Rotationsachse senkrecht auf der in Aufsicht gesehen bevorzugt kreisförmigen Vorrichtung.

Die vorliegende, erfindungsgemäße Vorrichtung weist eine Trägerplatteneinheit mit mindestens zwei Trägerplatten auf, einer ersten und einer zweiten Trägerplatte, gegebenenfalls können aber auch drei, vier, fünf, sechs oder mehr Trägerplatten vorgesehen sein.

In bevorzugter Ausführungsform weist die erfindungsgemäße Vorrichtung, insbesondere die Trägerplatteneinheit, insbesondere die erste und zweite Trägerplatte, in Aufsicht gesehen, die Form eines Kreises auf. Insbesondere ist sie als im Wesentlichen planarer, das heißt flacher dreidimensionaler Körper ausgebildet. Insbesondere weist die erfindungsgemäße Vorrichtung die Form eines flachen Zylinders, insbesondere einer Scheibe, also einer Disk, auf. Die horizontal flächige Ausdehnung der erfindungsgemäß bevorzugt in flacher, dreidimensionaler Form vorliegenden erfindungsgemäßen Vorrichtung ist daher wesentlich größer als deren vertikale Ausdehnung, wobei die horizontale Ausdehnung der erfindungsgemäßen Vorrichtung, insbesondere Trägerplatte, sowie der in ihr vorhandenen Strukturen, wie Öffnungen, Kanäle und Kammern, als Länge und Breite und die vertikale Ausdehnung als Höhe bezeichnet wird. Die durch die Länge und Breite (Länge ist die horizontale Ausdehnung, die größer als die Breite ist) aufgespannte Fläche der erfindungsgemäßen Vorrichtung, insbesondere der ersten und zweiten Trägerplatte, oder von deren Strukturen, wird als horizontale Fläche der erfindungsgemäßen Vorrichtung, Trägerplatte oder Strukturen bezeichnet.

In besonders bevorzugter Ausführungsform weist die erfindungsgemäße Vorrichtung, insbesondere die Trägerplatteneinheit, in Aufsicht gesehen, also eine Kreisform auf, wobei in bevorzugter Ausführungsform die Rotationsachse senkrecht auf dem Kreis stehend durch den Mittelpunkt der, in Aufsicht gesehen, kreisförmigen, insbesondere scheibenförmig vorliegenden, Vorrichtung verläuft und wobei die Distanz zwischen dem geometrischen Mittelpunkt des Kreises und dem Außenumfang des Kreises als Radius r bezeichnet wird.

In bevorzugter Ausführungsform der Erfindung beträgt der Durchmesser der, bevorzugt scheibenförmig vorliegenden, erfindungsgemäßen Vorrichtung 1 bis 80 cm, insbesondere 5 bis 64 cm, insbesondere 5 bis 30 cm, bevorzugt 7 bis 20 cm, bevorzugt 15 cm, besonders bevorzugt 10 cm.

In einer weiteren bevorzugten Ausführungsform der vorliegenden Erfindung weist die erfindungsgemäße Vorrichtung, insbesondere die Trägerplatteneinheit, insbesondere die erste und zweite Trägerplatte, in Aufsicht gesehen, die Form eines Rechtecks, bevorzugt eines Quadrats, auf. Erfindungsgemäß denkbar sind jedoch auch andere Formen, die eine zentrale Rotationsachse aufweisen. Insbesondere ist die erfindungsgemäße Vorrichtung, insbesondere die Trägerplatteneinheit, insbesondere die erste und zweite Trägerplatte, als im Wesentlichen planarer, das heißt flacher dreidimensionaler Körper ausgebildet. Insbesondere weist die erfindungsgemäße Vorrichtung die Form eines flachen Quaders, auf. Die horizontal flächige Ausdehnung der erfindungsgemäß bevorzugt in flacher, dreidimensionaler Form vorliegenden erfindungsgemäßen Vorrichtung ist dabei bevorzugt wesentlich größer als deren vertikale Ausdehnung, wobei die horizontale Ausdehnung der erfindungsgemäßen Vorrichtung, insbesondere Trägerplatte, sowie der in ihr vorhandenen Strukturen, wie Öffnungen, Kanäle und Kammern, als Länge und Breite und die vertikale Ausdehnung als Höhe bezeichnet wird. Die durch die Länge und Breite (Länge ist die horizontale Ausdehnung, die größer als die Breite ist) aufgespannte Fläche der erfindungsgemäßen Vorrichtung, insbesondere der ersten und zweiten Trägerplatte, oder von deren Strukturen, wird als horizontale Fläche der erfindungsgemäßen Vorrichtung, Trägerplatte oder Strukturen bezeichnet.

In besonders bevorzugter Ausführungsform der vorliegenden Erfindung ist die erfindungsgemäße Vorrichtung, insbesondere die Trägerplatteneinheit, insbesondere die erste und zweite Trägerplatte, als Mikrotiterplatte, insbesondere in Form einer Mikrotiterplatte, ausgestaltet.

In einer weiteren bevorzugten Ausführungsform der vorliegenden Erfindung ist die erfindungsgemäße Vorrichtung, insbesondere die Trägerplatteneinheit, insbesondere die erste und zweite Trägerplatte, als Mikrotiterplatte, insbesondere in Form einer Mikrotiterplatte, ausgestaltet, wobei die mindestens eine proximal zu der Rotationsachse angeordnete Zugangsöffnung an einer definierten Position der erfindungsgemäßen Vorrichtung angeordnet ist, bevorzugt alle proximal zu der Rotationsachse angeordneten Zugangsöffnungen an definierten Positionen der erfindungsgemäßen Vorrichtung angeordnet sind. Bevorzugt befinden sich die mindestens eine proximal zu der Rotationsachse angeordnete Zugangsöffnung auf einer der Lochpositionen einer Mikrotiterplatte, bevorzugt einer 96-well-Platte, bevorzugt einer 384-well-Platte. Bevorzugt befinden sich alle proximal zu der Rotationsachse angeordneten Zugangsöffnungen auf Lochpositionen einer Mikrotiterplatte, bevorzugt einer 96-well-Platte, bevorzugt einer 384-well-Platte. Gemäß dieser Ausführungsform ist vorteilhaftweise eine automatisierte Befüllung der mindestens einen proximal zu der Rotationsachse angeordneten Zugangsöffnung mittels eines Pipettierroboters möglich.

In besonders bevorzugter Ausführungsform ist die vorliegende Vorrichtung für die Kultivierung von Zellen auch eine Vorrichtung für die Herstellung von Zellverbänden. Unter dem Begriff Zellverband wird vorliegend auch ein Gewebe, insbesondere mikroskopisches Gewebe, Gewebeverband, Organ oder Organäquivalent verstanden. Insbesondere wird unter dem Begriff Zellverband auch ein Zellverband mit hoher Zelldichte und dreidimensionaler Ausdehnung verstanden.

Insbesondere stellt die erfindungsgemäße Vorrichtung eine mikrofluidische Vorrichtung, insbesondere ein Zellkultursystem, insbesondere ein mikrofluidisches Zellkultursystem, insbesondere ein Organ-on-a-disk System, insbesondere ein mikrofluidisches Organ-on-a-disk System, dar.

Im Zusammenhang mit der vorliegenden Erfindung wird unter einem Zentralbereich der Vorrichtung ein die zentrale Rotationsachse unmittelbarer umgebender Bereich verstanden. Dieser kann bevorzugt eine Fläche innerhalb eines bevorzugt kreisförmigen Umfangs mit vom geometrischen Mittelpunkt der, in Aufsicht gesehen, kreisförmigen Vorrichtung ausgehenden Radius r_{z} (Radius des Zentralbereichs) = 1/4 r bis 1/10 r der Trägerplatteneinheit sein. Insbesondere ist der Radius des Zentralbereichs (r_{z}) der Trägerplatteneinheit mindestens r_{z}=1/10 r der Trägerplatteneinheit und höchstens r_{z}=1/4 r der Trägerplatteneinheit.

In bevorzugter Ausführungsform weist der Zentralbereich der erfindungsgemäßen Vorrichtung mindestens eine Verbindungsvorrichtung für eine eine Rotation der Vorrichtung ermöglichende Drehvorrichtung, zum Beispiel einen externen Drehantrieb, zum Beispiel einen Motor, auf. Die Verbindungsvorrichtung ist insbesondere mindestens eine Durchgangsöffnung, zum Beispiel 2, 3, 4, 5, oder 6 Durchgangsöffnungen, oder mindestens eine Verankerungsvorrichtung, zum Beispiel 2, 3 oder 4 Verankerungsvorrichtungen. Die Verbindungsvorrichtung, insbesondere die mindestens eine Durchgangsöffnung oder mindestens eine Verankerungsvorrichtung, dient dem Befestigen der erfindungsgemäßen Vorrichtung an eine eine Rotation ermöglichende Drehvorrichtung, die die erfindungsgemäße Vorrichtung in Rotation versetzen kann.

Bevorzugterweise ist die Durchgangsöffnung ein Loch, Nabe oder Gewinde oder ein Teil davon. Die Verankerungsvorrichtung ist bevorzugt eine Steck-, Klapp-, Rast-, Schnapp- oder Klemmverbindungsvorrichtung oder ein Teil davon.

Es kann auch vorgesehen sein, dass die Vorrichtung mindestens eine, vorzugsweise peripher angeordnete, Arretierungsvorrichtung für eine Drehvorrichtung aufweist. Diese Arretierungsvorrichtung kann beispielsweise mindestens eine Klammer sein, die die erfindungsgemäße Vorrichtung an deren Rand, insbesondere äußersten Rand, einspannt.

Es kann erfindungsgemäß vorgesehen sein, dass die Vorrichtung mithilfe der mindestens einen Verbindungsvorrichtung des Zentralbereichs und/oder mithilfe der mindestens einen peripher angeordneten Arretierungsvorrichtung an der Drehvorrichtung befestigt wird. Weiterhin ist es erfindungsgemäß möglich, dass die erfindungsgemäße Vorrichtung selbst keine Verbindungs- oder Arretierungsvorrichtung aufweist, sondern die Drehvorrichtung eine Fixierungsvorrichtung für die erfindungsgemäße Vorrichtung aufweist. Diese Fixierungsvorrichtung kann eine dem Umfang der erfindungsgemäßen Vorrichtung angepassten Aufnahme der Drehvorrichtung sein.

In bevorzugter Ausführungsform der Vorrichtung ist der mindestens eine Kanal, der die mindestens eine Zugangsöffnung mit der mindestens einen Kultivierungskammer verbindet, ein unverzweigter Kanal. Erfindungsgemäß bevorzugt kann der Kanal jedoch auch ein einfach, mehrfach oder vielfach verzweigter Kanal sein und verbindet mindestens eine Zugangsöffnung mit mindestens zwei Kultivierungskammern, insbesondere in paralleler Anordnung der Kanäle und Kultivierungskammern zueinander. Dies ermöglicht die parallele, gleichzeitige Befüllung mehrerer Kultivierungskammern mit Zellen durch eine Zugangsöffnung. In bevorzugter Ausführungsform verbindet der verzweigte Kanal mindestens eine Zugangsöffnung mit mindestens drei Kultivierungskammern, bevorzugt mit mindestens vier Kultivierungskammern, bevorzugt mit mindestens fünf Kultivierungskammern, bevorzugt mit mindestens sechs Kultivierungskammern, bevorzugt mit mindestens sieben Kultivierungskammern, bevorzugt mit mindestens acht Kultivierungskammern, bevorzugt mit mindestens neun Kultivierungskammern, bevorzugt mit mindestens zehn Kultivierungskammern, bevorzugt mit mindestens 11 Kultivierungskammern, bevorzugt mit mindestens 12 Kultivierungskammern, bevorzugt mit mindestens 13 Kultivierungskammern, bevorzugt mit mindestens 14 Kultivierungskammern, bevorzugt mit mindestens 15 Kultivierungskammern, bevorzugt mit mindestens 20 Kultivierungskammern, bevorzugt mit mindestens 25 Kultivierungskammern, bevorzugt mit mindestens 30 Kultivierungskammern, bevorzugt mit mindestens 35 Kultivierungskammern, bevorzugt mit mindestens 40 Kultivierungskammern, bevorzugt mit mindestens 45 Kultivierungskammern, bevorzugt mit mindestens 50 Kultivierungskammern, bevorzugt mit mindestens 60 Kultivierungskammern, bevorzugt mit mindestens 70 Kultivierungskammern, bevorzugt mit mindestens 80 Kultivierungskammern, bevorzugt mit mindestens 90 Kultivierungskammern, bevorzugt mit mindestens 100 Kultivierungskammern, vorzugsweise jeweils in paralleler Anordnung der Kanäle und Kultivierungskammern zueinander.

In bevorzugter Ausführungsform ist der mindestens eine Kanal ein verzweigter oder unverzweigter Kanal, der die mindestens eine Zugangsöffnung mit mindestens zwei Kultivierungskammern, bevorzugt mit mindestens drei Kultivierungskammern, bevorzugt mit mindestens vier Kultivierungskammern, bevorzugt mit mindestens fünf Kultivierungskammern, bevorzugt mit mindestens sechs Kultivierungskammern, bevorzugt mit mindestens sieben Kultivierungskammern, bevorzugt mit mindestens acht Kultivierungskammern, bevorzugt mit mindestens neun Kultivierungskammern, bevorzugt mit mindestens zehn Kultivierungskammern, bevorzugt mit mindestens 11 Kultivierungskammern, bevorzugt mit mindestens 12 Kultivierungskammern, bevorzugt mit mindestens 13 Kultivierungskammern, bevorzugt mit mindestens 14 Kultivierungskammern, bevorzugt mit mindestens 15 Kultivierungskammern, bevorzugt mit mindestens 20 Kultivierungskammern, bevorzugt mit mindestens 25 Kultivierungskammern, bevorzugt mit mindestens 30 Kultivierungskammern, bevorzugt mit mindestens 35 Kultivierungskammern, bevorzugt mit mindestens 40 Kultivierungskammern, bevorzugt mit mindestens 45 Kultivierungskammern, bevorzugt mit mindestens 50 Kultivierungskammern, bevorzugt mit mindestens 60 Kultivierungskammern, bevorzugt mit mindestens 70 Kultivierungskammern, bevorzugt mit mindestens 80 Kultivierungskammern, bevorzugt mit mindestens 90 Kultivierungskammern, bevorzugt mit mindestens 100 Kultivierungskammern, verbindet, vorzugsweise jeweils in paralleler Anordnung der Kanäle und Kultivierungskammern zueinander.

Der mindestens eine Kanal, der die mindestens eine Zugangsöffnung mit der mindestens einen Kultivierungskammer, bevorzugt mit den mindestens zwei Kultivierungskammern verbindet, kann in bevorzugter Ausführungsform eine Breite von 10 bis 1000 µm, bevorzugt 10 bis 800 µm, bevorzugt 10 bis 600 µm, bevorzugt 10 bis 500 µm, bevorzugt 10 bis 400 µm, insbesondere 50 bis 150 µm, insbesondere 70 µm aufweisen. In besonders bevorzugter Ausführungsform weist der mindestens eine Kanal eine Höhe von 10 bis 400 µm, insbesondere 50 bis 150 µm, insbesondere 70 µm auf. In besonders bevorzugter Ausführungsform kann der mindestens eine Kanal verzweigt sein, vorzugsweise in radialer Richtung, und insbesondere 0 bis 10, vorzugsweise 1, 2, 3, 4, 5 oder 6 Verzweigungen aufweisen. Besonders bevorzugt kann die Kanalhöhe und/oder die Kanalbreite mit der Anzahl der Verzweigungen zunehmen.

In besonders bevorzugter Ausführungsform der vorliegenden Erfindung weist der Kanal mindestens über einen Teil seiner Länge mindestens zwei, bevorzugt mindestens drei, bevorzugt mindestens vier, bevorzugt mindestens fünf, bevorzugt mindestens sechs, bevorzugt mindestens sieben, bevorzugt mindestens acht, bevorzugt mindestens neun, bevorzugt mindestens zehn bevorzugt mit mindestens 11, bevorzugt mit mindestens 12, bevorzugt mit mindestens 13, bevorzugt mit mindestens 14, bevorzugt mit mindestens 15, bevorzugt mit mindestens 20, bevorzugt mit mindestens 25, bevorzugt mit mindestens 30, bevorzugt mit mindestens 35, bevorzugt mit mindestens 40, bevorzugt mit mindestens 45, bevorzugt mit mindestens 50, bevorzugt mit mindestens 60, bevorzugt mit mindestens 70, bevorzugt mit mindestens 80, bevorzugt mit mindestens 90, bevorzugt mit mindestens 100, unmittelbar an den Kanal angrenzende Kultivierungskammern auf.

In besonders bevorzugter Ausführungsform der vorliegenden Erfindung sind die unmittelbar an den Kanal angrenzenden Kultivierungskammern mindestens über einen Teil der Länge des Kanals parallel zueinander angeordnet.

In einer weiteren bevorzugten Ausführungsform der vorliegenden Erfindung ist der mindestens eine Kanal mindestens über einen Teil seiner Länge gekrümmt. Bevorzugt weist der mindestens eine Kanal mindestens über einen Teil seiner Länge eine statische Krümmung auf. Bevorzugt weist der mindestens eine Kanal mindestens über einen Teil seiner Länge eine winkelanhängige Krümmung auf. In besonders bevorzugter Ausführungsform ist der mindestens eine Kanal mindestens über einen Teil seiner Länge um einen definierten Winkel zur Richtung der durch Rotation erzeugten Zentrifugalkraft gekrümmt. In besonders bevorzugter Ausführungsform der vorliegenden Erfindung nimmt die Krümmung des mindestens einen Kanals von der mindestens einen proximal zu der Rotationsachse angeordneten Zugangsöffnung in Richtung der mindestens einen distal zu der Rotationsachse angeordneten Kultivierungskammer ab. Bevorzugt nimmt die Krümmung des mindestens einen Kanals von der mindestens einen proximal zu der Rotationsachse angeordneten Zugangsöffnung in Richtung der mindestens einen distal zu der Rotationsachse angeordneten Kultivierungskammer stetig ab.

In besonders bevorzugter Ausführungsform weist die mindestens eine Zugangsöffnung einen Durchmesser von 0,2 bis 20 mm, bevorzugt 0,5 bis 10 mm, vorzugsweise 1 bis 8 mm, insbesondere 3 mm auf.

In einer besonders bevorzugten Ausführungsform der vorliegenden Erfindung weist die Vorrichtung zwischen der mindestens einen proximal zu der Rotationsachse angeordneten Zugangsöffnung und dem mindestens einen Kanal einen Verbindungsbereich auf. Bevorzugt verläuft der Verbindungsbereich schräg abfallend von der mindestens einen proximal zu der Rotationsachse angeordneten Zugangsöffnung zu dem mindestens einen Kanal. Mit Hilfe eines solchen schräg abfallend verlaufenden Verbindungsbereichs ist es vorteilhafterweise möglich, einen verbesserten Fluss der durch die mindestens eine Zugangsöffnung in die Trägerplatteneinheit eingebrachten Zellen, insbesondere die Zellsuspension, gegebenenfalls auch Zellkulturmedium, von der mindestens einen proximal zu der Rotationsachse angeordneten Zugangsöffnung über den Verbindungsbereich zu dem mindestens einen Kanal zu gewährleisten.

In einer bevorzugten Ausführungsform ist die mindestens eine Zugangsöffnung als Ladekammer ausgestaltet, die mindestens zwei Zugangsöffnungen aufweist. Durch die Ausgestaltung der mindestens einen Zugangsöffnung als Ladekammer ist es vorteilhafterweise möglich, die Ladeeffizienz der erfindungsgemäßen Vorrichtung zu verbessern. Dabei besitzt die Ladekammer mindestens zwei Zugangsöffnungen über die bevorzugt eine Strömung innerhalb der Ladekammer erzeugt werden kann. Durch die Ladekammer ist es möglich, die Anzahl der bei Rotation der erfindungsgemäßen Vorrichtung tatsächlich in die mindestens eine Kultivierungskammer transportierten Zellen zu erhöhen.

In besonders bevorzugter Ausführungsform weist die mindestens eine Kultivierungskammer in runder Form einen horizontalen Durchmesser von 0,05 bis 10 mm, bevorzugt 0,1 bis 9 mm, vorzugsweise bis 0,2 bis 8 mm, insbesondere 2 mm auf. In besonders bevorzugter Ausführungsform beträgt der radiale Abstand (r₂) der Kultivierungskammern zum Mittelpunkt der Vorrichtung 1 bis 30 cm, bevorzugt 1 bis 20 cm, vorzugsweise 2 bis 15 cm, insbesondere 4,5 cm. Über den radialen Abstand (r₂) der Kultivierungskammern zum Mittelpunkt der Vorrichtung kann vorteilhafterweise das Verhältnis von a(ω) eingestellt werden.

In besonders bevorzugter Ausführungsform weist die mindestens eine Kultivierungskammer in Hantelform einen Stegabstand von 0,3 bis 10 mm, bevorzugt 1 mm auf. Die Stegbreite beträgt 50 µm bis 500 µm, bevorzugt 150 µm. Die beiden Enden weisen eine Aufweitung senkrecht zum Steg von 100 µm bis 2 mm, bevorzugt 550 µm auf. Die Aufweitung erfolgt abrupt in 90° oder nimmt trapezförmig in einem beliebigen Winkel zwischen 0° und 90°, bevorzugt 42° zu. Die Enden besitzen eine radiale Ausdehnung von 100 µm bis 1 mm, bevorzugt 300 µm. Um eine gleichmäßige Befüllung der Kammer in Hantelform zu gewährleisten ist in bevorzugter Ausführungsform die distal zur Rotationsachse ausgerichtete Seite abgerundet.

In besonders bevorzugter Ausführungsform weist die mindestens eine Kultivierungskammer in rechteckiger Form eine Breite von 0,05 bis 10 mm, bevorzugt 500 µm, sowie eine Höhe von 0,05 bis 10 mm, bevorzugt 1500 µm auf.

In besonders bevorzugter Ausführungsform beträgt die Höhe der Trägerplatteneinheit, insbesondere der ersten Trägerplatte, 0,8 bis 20 mm, bevorzugt 1,5 bis 4 mm, insbesondere 1,7 bis 2,5 mm, insbesondere 2 mm. Bevorzugt sollte die Höhe der ersten Trägerplatte der Kanalhöhe plus mindestens einen halben Millimeter, bevorzugt einen Millimeter, entsprechen, um eine ausreichende Stabilität der Vorrichtung zu gewährleisten.

Die mindestens eine Kultivierungskammer kann vorzugsweise rund, elliptisch, rechteckig, trapezförmig, hantelförmig, in Form eines Kreissegments oder Kreissektors, sowie Teile oder Kombinationen der genannten Formen ausgebildet sein.

In besonders bevorzugter Ausführungsform ist die Trägerplatteneinheit einstückig ausgeführt.

In besonders bevorzugter Ausführungsform ist die erfindungsgemäße Vorrichtung die Trägerplatteneinheit. In besonders bevorzugter Ausführungsform ist die erfindungsgemäße Vorrichtung, insbesondere die Trägerplatteneinheit, eine Trägerplatte, insbesondere die erste Trägerplatte, also die Trägerplatte, in der die Kultivierung von Zellen stattfindet und die durch die Anwesenheit der mindestens einen Zugangsöffnung, der mindestens einen Kultivierungskammer und dem mindestens einen die mindestens eine Zugangsöffnung und die mindestens eine Kultivierungskammer verbindenden Kanal ausgezeichnet ist.

Die in der Trägerplatteneinheit, insbesondere der ersten Trägerplatte, ausgebildete mindestens eine Zugangsöffnung, mindestens eine Kultivierungskammer und der mindestens eine die mindestens eine Zugangsöffnung und die mindestens eine Kultivierungskammer verbindende Kanal sind in der Trägerplatteneinheit, insbesondere in der ersten Trägerplatte, integriert oder an ihrer Oberfläche angeordnet und, in bevorzugter Ausführungsform, in einer horizontalen Fläche der Trägerplatte vollständig oder teilweise offen, das heißt weisen jeweils Boden und Wandteile in der Trägerplatteneinheit, insbesondere in der Trägerplatte und eine Öffnung in der horizontalen Fläche auf.

Im Zusammenhang mit der vorliegenden Erfindung wird die horizontale Fläche der Trägerplatteneinheit, insbesondere der ersten Trägerplatte, die die Öffnung der mindestens einen Kultivierungskammer aufweist als obere oder nach oben hin gewandte Fläche der ersten Trägerplatte oder der Trägerplatteneinheit bezeichnet.

In besonders bevorzugter Ausführungsform kann die Trägerplatteneinheit, insbesondere die erste Trägerplatte, durch einen, vorzugsweise reversibel aufbringbaren Deckel, oder eine Schicht, zum Beispiel eine PDMS-Schicht, nach oben hin gewandt vollständig oder teilweise abgeschlossen sein, sodass zumindest die mindestens eine Kultivierungskammer und der mindestens eine Kanal fluiddicht nach außen verschlossen sind. Die mindestens eine Zugangsöffnung kann bevorzugt reversibel verschließbar ausgestaltet sein, um eine Befüllung mit Zellen und/oder Kulturmedium und anschließendem Verschließen zu ermöglichen. Die mindestens eine Zugangsöffnung kann gegebenenfalls auch auf der zu der oberen horizontalen Fläche der Trägcrplattcncinhcit, insbesondere ersten Trägerplatte, entgegengesetzt angeordneten unteren horizontalen Fläche angeordnet sein.

In einer bevorzugten Ausführungsform umfasst die Trägerplatteneinheit, insbesondere besteht die Trägerplatteneinheit aus, mindestens zwei Trägerplatten, insbesondere einer ersten und einer zweiten Trägerplatte, die als separate Bauteile vorliegen und nach reversiblem oder irreversiblem Zusammenfügen in bevorzugter Ausführungsform übereinander, vorzugsweise deckungsgleich miteinander, angeordnet eine Trägerplatteneinheit bilden. In bevorzugter Ausführungsform umfasst die erfindungsgemäße Vorrichtung, insbesondere die Trägerplatteneinheit, also mindestens eine erste Trägerplatte, insbesondere für die Kultivierung von Zellen, und mindestens eine nach Zusammenfügen beider Trägerplatten darüber oder darunter angeordnete und mit ihr, bevorzugt nach außen hin fluiddicht, verbundene zweite Trägerplatte, insbesondere für die Versorgung der sich in der Kultivierungskammer befindenden Zellen mit Kulturmedium.

In dieser Ausführungsform weist die erste Trägerplatte die mindestens eine Zugangsöffnung, die mindestens eine Kultivierungskammer und den mindestens einen, die mindestens eine Zugangsöffnung und die mindestens eine Kultivierungskammer verbindenden Kanal auf. Erfindungsgemäß bevorzugt weist die auf der ersten Trägerplatte, bevorzugt nach außen hin fluiddicht, angeordnete zweite Trägerplatte mindestens eine, von außerhalb der Trägerplatteneinheit zugängliche Medienöffnung, mindestens eine Medienkammer und mindestens einen die mindestens eine Medienöffnung mit der mindestens einen Medienkammer verbindenden Medienkanal auf, wobei zumindest die mindestens eine Medienkammer eine Öffnung aufweist, insbesondere eine Öffnung, die eine fluidische Verbindung zu der nach Zusammenfügen beider Trägerplatten unterhalb oder oberhalb der zweiten Trägerplatte angeordneten mindestens einen Kultivierungskammer der ersten Trägerplatte ermöglicht, und wobei die Medienöffnung als Einlass oder Auslass für Medien, insbesondere als eine von außerhalb der Trägerplatteneinheit zugängliche Öffnung ausgeführt ist. Die mindestens eine Medienöffnung ist bevorzugt auf der der ersten Trägerplatte abgewandten oberen horizontalen Fläche der zweiten Trägerplatte angeordnet. Die mindestens eine Medienöffnung, die mindestens eine Medienkammer und der mindestens eine die mindestens eine Medienöffnung und die mindestens eine Medienkammer verbindende Medienkanal sind in der zweiten Trägerplatte integriert oder an ihrer Oberfläche angeordnet, und, in bevorzugter Ausführungsform in einer horizontalen Fläche der Trägerplatte vollständig oder teilweise offen, das heißt weisen jeweils Boden und Wandtcilc in der Trägcrplattc und eine Öffnung in der horizontalen Fläche auf.

In bevorzugter Ausführungsform weist die zweite Trägerplatte mindestens zwei Medienöffnungen auf, insbesondere mindestens einen Medieneinlass und mindestens einen Medienauslass, die über mindestens einen Medienkanal und mindestens eine Medienkammer miteinander verbunden sind. Bevorzugt verbindet der mindestens eine Medienkanal mindestens zwei Medienöffnungen mit mindestens einer, vorzugsweise mindestens zwei, Medienkammern.

In einer besonders bevorzugten Ausführungsform kann die mindestens eine Zugangsöffnung der Trägerplatteneinheit, insbesondere der ersten Trägerplatte, in der von der zweiten Trägerplatte abgewandten nach unten weisenden horizontalen Fläche der ersten Trägerplatte lokalisiert sein. Der mindestens eine Medienkanal kann in bevorzugter Ausführungsform, ebenso wie die mindestens eine Medienkammer, in der der ersten Trägerplatte, nach Zusammenfügen der beiden Trägerplatten, zugewandten unteren horizontalen Fläche hin geöffnet vorliegen und nach Zusammenfügen mit der ersten Trägerplatte bevorzugt in fluidischer Verbindung mit dem mindestens einen darunterliegenden Kanal und der mindestens einen darunter liegenden Kultivierungskammer stehen.

In besonderes bevorzugter Ausführungsform kann die erste Trägerplatte allerdings auch über der zweiten Trägerplatte angeordnet sein oder gegebenenfalls zwischen zwei Medienöffnungen, Medienkanäle und Medienkammern aufweisenden Trägerplatten angeordnet sein.

Erfindungsgemäß bevorzugt kann es auch vorgesehen sein, dass lediglich die Medienkammer der zweiten Trägerplatte mit der Kultivierungskammer der ersten Trägerplatte in fluidischer Verbindung steht und die Kanäle der ersten Trägerplatte und die Medienkanäle der zweiten Trägerplatte jeweils fluiddicht vorliegen und gegebenenfalls auch nicht miteinander überlappend ausgestaltet sind.

Sofern in erfindungsgemäß bevorzugter Ausführungsform mindestens zwei Trägerplatten, insbesondere eine erste und eine zweite Trägerplatte, vorgesehen sind, deckt die zweite Trägerplatte nach Zusammenfügen mit der ersten Trägerplatte die in ihrer oberen Fläche vorhandenen nach oben gerichteten Öffnungen der mindestens einen Kultivierungskammer und des mindestens einen Kanals sowie gegebenenfalls der mindestens einen Zugangsöffnung nach oben hin, vorzugsweise fluiddicht, ab, wobei die mindestens eine Kultivierungskammer der ersten Trägerplatte und die mindestens eine Medienkammer der zweiten Trägerplatte miteinander fluidisch in Verbindung stehen.

In bevorzugter Ausführungsform ist vorgesehen, in der mindestens einen zweiten Trägerplatte einen Durchlass für die Zugangsöffnung der nach Zusammenfügen beider Trägerplatten unterhalb der zweiten Trägerplatte angeordneten ersten Trägerplatte auszuführen. Dieser Durchlass kann reversibel verschließbar ausgestaltet sein.

Es ist erfindungsgemäß also besonders bevorzugt, dass die mindestens eine Kultivierungskammer der ersten Trägerplatte und die mindestens eine Medienkammer der zweiten Trägerplatte überlappend zueinander angeordnet sind und in fluidischer Verbindung miteinander stehen. Die sich in den Kultivierungskammern befindenden Zellen können somit über die Medienöffnung, den Medienkanal und die Medienkammer mit Nährstoffen versorgt werden, insbesondere durch die Zugabe von Zellkulturmedium. Weiterhin können bevorzugt über die in der zweiten Trägerplatte ausgebildeten Medienöffnungen, Medienkanäle und Medienkammern therapeutische, pharmazeutische oder kosmetische Präparate und Substanzen an die Zellen verabreicht werden. Dies ermöglicht vorteilhafterweise eine einfache Testung der therapeutischen Wirksamkeit oder Toxizität von Substanzen an den Zellen oder Gewebeverbänden in den Kultivierungskammern.

In besonders bevorzugter Ausführungsform kann also vorgesehen sein, dass die erste Trägerplatte mindestens eine proximal zu der Rotationsachse angeordnete Zugangsöffnung, mindestens eine distal zu der Rotationsachse angeordnete Kultivierungskammer und mindestens einen die mindestens eine Zugangsöffnung mit der mindestens einen Kultivierungskammer verbindenden Kanal aufweist und diese erste Trägerplatte der Kultivierung von Zellen, insbesondere der Herstellung von Zellverbänden, insbesondere Geweben, dient und die auf der ersten Trägerplatte, vorzugsweise fluiddicht, angeordnete zweite Trägerplatte der Versorgung der kultivierten Zellen dient und insbesondere mindestens eine Medienöffnung, mindestens eine Medienkammer und mindestens einen die mindestens eine Medienöffnung mit der mindestens einen Medienkammer verbindenden Medienkanal aufweist, und wobei sowohl die Medienöffnung als auch die Zugangsöffnung als von außerhalb der Trägerplatteneinheit zugängliche Öffnungen ausgeführt sind und zumindest die mindestens eine Kultivierungskammer mit der mindestens einen Medienkammer überlappt und fluidisch miteinander in Verbindung stehen, sodass eine Versorgung der Kultivierungskammer mit Medium durch die Medienkammer ermöglicht wird. In bevorzugter Ausführungsform kann auch der mindestens eine Medienkanal überlappend und in fluidischer Verbindung stehend mit dem mindestens einen, die mindestens eine Zugangsöffnung mit der mindestens einen Kultivierungskammer verbindenden Kanal ausgeführt sein.

In bevorzugter Ausführungsform ist der mindestens eine Medienkanal der zweiten Trägerplatte, der die mindestens eine Medienöffnung mit der mindestens einen Medienkammer verbindet, ein unverzweigter Medienkanal. Erfindungsgemäß bevorzugt kann der Medienkanal auch ein einfach, mehrfach oder vielfach verzweigter Medienkanal sein und verbindet mindestens eine Medienöffnung, vorzugsweise mindestens zwei Medienöffnungen, parallel oder seriell mit mindestens zwei Medienkammern. Dies ermöglicht die parallele Befüllung mehrerer Medienkammern mit Zellkulturmedium, pharmazeutischen oder kosmetischen Substanzen oder ähnlichem durch eine Medienöffnung. In bevorzugter Ausführungsform verbindet der verzweigte Medienkanal mindestens eine Medienöffnung mit mindestens drei Medienkammern, bevorzugt mit mindestens vier Medienkammern, bevorzugt mit mindestens fünf Medienkammern, bevorzugt mit mindestens sechs Medienkammern, bevorzugt mit mindestens sieben Medienkammern, bevorzugt mit mindestens acht Medienkammern, bevorzugt mit mindestens neun Medienkammern, bevorzugt mit mindestens zehn Medienkammern, bevorzugt mit mindestens 11 Medienkammern, bevorzugt mit mindestens 12 Medienkammern, bevorzugt mit mindestens 13 Medienkammern, bevorzugt mit mindestens 14 Medienkammern, bevorzugt mit mindestens 15 Medienkammern, bevorzugt mit mindestens 20 Medienkammern, bevorzugt mit mindestens 25 Medienkammern, bevorzugt mit mindestens 30 Medienkammern, bevorzugt mit mindestens 35 Medienkammern, bevorzugt mit mindestens 40 Medienkammern, bevorzugt mit mindestens 45 Medienkammern, bevorzugt mit mindestens 50 Medienkammern, bevorzugt mit mindestens 60 Medienkammern, bevorzugt mit mindestens 70 Medienkammern, bevorzugt mit mindestens 80 Medienkammern, bevorzugt mit mindestens 90 Medienkammern, bevorzugt mit mindestens 100 Medienkammern, jeweils in paralleler Anordnung der Medienkanäle und Medienkammern zueinander.

In besonders bevorzugter Ausführungsform weist der mindestens eine Medienkanal der zweiten Trägerplatte, der die mindestens eine Medienöffnung mit der mindestens einen Medienkammer verbindet, keinen senkrecht zur Richtung der durch Rotation erzeugten Zentrifugalkraft verlaufenden Abschnitt auf. Gemäß dieser Ausführungsform können vorteilhafterweise Lufteinschlüsse verhindert werden und eine optimale Medienversorgung durch Rotation gewährleistet werden. In einer bevorzugten Ausführungsform kann auch vorgesehen sein, dass der mindestens eine Medienkanal mindestens eine Medienöffnung mit mindestens zwei oder mehr Medienkammern, die in Reihe angeordnet sind, verbindet. Das heißt, der mindestens eine Medienkanal verbindet die mindestens zwei Medienkammern seriell. Weiterhin kann vorgesehen sein, dass der mindestens eine Medienkanal mindestens zwei Medienöffnungen mit mindestens einer, bevorzugt mindestens zwei, drei, vier, fünf, sechs, sieben, acht, neun oder zehn Medienkammern, die in Reihe angeordnet sind, verbindet. Hierbei kann vorgesehen sein, dass eine der mindestens zwei Medienöffnungen als Medieneinlass und eine zweite als Medienauslass fungiert, sodass das Medium, insbesondere das Kulturmedium, den Medienkanal durchströmen, insbesondere zirkulieren, kann. Bevorzugt durchströmt das Kulturmedium den Medienkanal kontinuierlich oder pulsativ. Dabei kann weiterhin vorgesehen sein, dass Pumpen, Druckgradienten oder ähnliches vorliegen oder integriert sind oder die Rotation der Vorrichtung genutzt wird, um einen Fluss des Mediums zu ermöglichen. In diesen Ausführungsformen verbindet der mindestens eine Medienkanal zwar mindestens zwei Medienöffnungen mit mindestens zwei oder mehr Medienkammern, ist aber kein verzweigter Medienkanal.

Es kann erfindungsgemäß ebenfalls vorgesehen sein, dass eine zweite Trägerplatte verzweigte Medienkanäle, die mindestens eine oder mindestens zwei Medienöffnungen mit mindestens zwei Medienkammern parallel verbinden und unverzweigte Medienkanäle, die mindestens eine oder mindestens zwei Medienöffnungen mit mindestens zwei Medienkammern seriell verbinden, aufweist.

In einer besonders bevorzugten Ausführungsform beträgt die Breite des mindestens einen Medienkanals 5 bis 600 µm, bevorzugt 10 bis 400 µm, vorzugsweise 50 bis 150 µm, insbesondere 70 µm. In besonders bevorzugter Ausführungsform beträgt die Höhe des mindestens einen Medienkanals 5 bis 600 µm, bevorzugt 10 bis 400 µm, vorzugsweise 50 bis 150 µm, insbesondere 70 µm. In besonders bevorzugter Ausführungsform kann der mindestens eine Medienkanal verzweigt sein, insbesondere von 0 bis 10, vorzugsweise 1, 2, 3, 4, 5 oder 6 Verzweigungen aufweisen. Besonders bevorzugt kann die Medienkanalhöhe und/oder die Medienkanalbreite mit der Anzahl der Verzweigungen zunehmen.

In besonders bevorzugter Ausführungsform beträgt der Durchmesser der mindestens einen Medienöffnung 0,5 bis 20 mm, bevorzugt 0,7 bis 10 mm, vorzugsweise 1 bis 8 mm. Im Zusammenspiel mit der Höhe der zweiten Trägerplatte ermöglicht dies das Einbringen von 1 bis 200 µl, bevorzugt 2 bis 100 µl, insbesondere 5 bis 50 µl Medienvolumen.

In besonders bevorzugter Ausführungsform weist die mindestens eine Medienkammer in runder Form einen horizontalen Durchmesser von 0,1 bis 10 mm, vorzugsweise 0,2 bis 8 mm, insbesondere 2 mm, auf. In besonders bevorzugter Ausführungsform beträgt der radiale Abstand der Medienkammern zum Mittelpunkt 1 bis 20 cm, vorzugsweise 2 bis 10 cm, insbesondere 4,5 cm.

Die mindestens eine Medienkammer kann in bevorzugter Ausführungsform der vorliegenden Erfindung rund, elliptisch, rechteckig, trapezförmig, hantelförmig, in Form eines Kreissegments oder Kreissektors, sowie Teile oder Kombinationen der genannten Formen ausgeführt sein.

Es kann erfindungsgemäß auch vorgesehen sein, dass die zweite Trägerplatte eine oder mehrere integrierte oder externe Pumpen aufweist, die die Mediumsversorgung gewährleisten.

In besonders bevorzugter Ausführungsform beträgt die Höhe der zweiten Trägerplatte 0,8 bis 20 mm, bevorzugt 1,5 bis 4 mm, insbesondere 1,7 bis 2,5 mm, insbesondere 2 mm. Bevorzugt sollte die Höhe der zweiten Trägerplatte der Medienkanalhöhe plus mindestens einen halben Millimeter, bevorzugt einen Millimeter, entsprechen, um eine ausreichende Stabilität der Vorrichtung zu gewährleisten.

Besonders bevorzugt ist zwischen der ersten Trägerplatte und der zweiten Trägerplatte mindestens eine Trennvorrichtung, insbesondere eine Membran, angeordnet. In bevorzugter Ausführungsform ist die Trennvorrichtung dünner als die erste und zweite Trägerplatte. Die Trennvorrichtung, insbesondere Membran, ist bevorzugt endothelähnlich und/oder porös. In bevorzugter Ausführung ermöglicht die Trennvorrichtung einen diffusiven Stoffaustausch zwischen der ersten und der zweiten Trägerplatte, insbesondere zwischen der mindestens einen Medienkammer der zweiten Trägerplatte und der mindestens eine Kultivierungskammer der ersten Trägerplatte. In bevorzugter Ausführung kann vorgesehen sein, dass jeweils eine Membran zwischen einer Kultivierungskammer und einer Medienkammer aufgebracht wird oder eine einzelne Membran in Trägerplattengröße oder ringförmig oder beliebig große Teilabschnitte davon in Breite der Medien- und/oder Kultivierungskammern verwendet wird. Bevorzugt ist die mindestens eine Trennvorrichtung, insbesondere Membran, aus PET (Polyethylenterephthalat), PC (Polycarbonat), Glas, PDMS (Polydimethylsiloxan) oder einem Negativ-Resist-Material, insbesondere Epoxid-Fotolack (SU-8 oder 1002F-50), hergestellt. Bevorzugt kann die Trennvorrichtung, insbesondere Membran auch aus Polyolefinen, Polystyrol, "Zellkultur behandeltem" Polystyrol, Polyalkylmethacrylat und Polyalkylacrylat, Polyacrylamid, Polycarbonat, Polyethylenglycol, Poly(N-isopropylacrylamid), Polyacrylonitril, Polyvinylacetat, Polyvinylalkoholen, Polyvinylchlorid, Polyoxymethylen, Polyamid, Polyimid, Polyurethan, Polyvinylidenfluorid (PVDF), Phenolen, Aminoepoxyharze, Polyester, Polyether, Polyethylenterephthalate (PET), Polyglycolsäuren (PGA) und anderen degradierbaren Polyestern, Poly-(p-phenylenterephthalamid), Polyphosphazen, Polypropylen, und Silikonelastomeren, sowie Copolymeren und Kombinationen davon hergestellt sein. In einer Ausführungsform kann die Membran auch degradierbar, insbesondere biodegradierbar sein.

Besonders bevorzugt weist die Trennvorrichtung eine präzise, definierte Struktur, biologische Kompatibilität sowie geringe Autofluoreszenz auf, um eine optische Untersuchung des kultivierten Gewebes zu ermöglichen. Vorteilhafterweise lässt sich die Trennvorrichtung je nach Anwendung sowie zu kultivierendem Zelltyp individualisieren, das heißt die Porengröße, Porosität und Anordnung der Poren können angepasst werden. Bevorzugt ist, dass die Poren kleiner als die verwendeten Zellen sind, um diese nicht aus den Kultivierungskammern zu spülen.

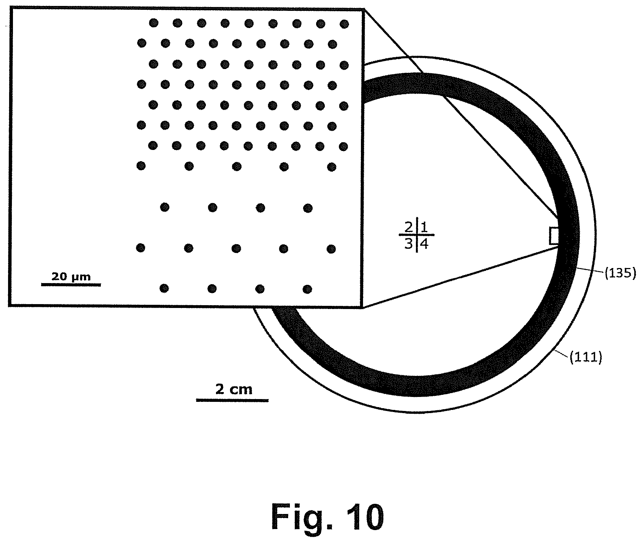

In bevorzugter Ausführungsform wird für die Trennvorrichtung ein hexagonales Gitter mit einer Porosität von 1-20 %, bevorzugt 5 bis 10 % und einer Porengröße von 1-5 µm, bevorzugt 4 µm verwendet (siehe Figur 10). Besonders bevorzugt befinden sich die durchlässigen Poren nur im Überlapp von Kultivierungskammer und Medienkammer, um die Diffusion nur in diesem Bereich zu gewährleisten.

In besonders bevorzugter Ausführungsform beträgt die Höhe der erfindungsgemäßen Vorrichtung, insbesondere der eine erste Trägerplatte, eine Trennvorrichtung und eine zweite Trägerplatte aufweisenden Vorrichtung 2 bis 40 mm, bevorzugt 2 bis 30 mm, bevorzugt 2 bis 20 mm, bevorzugt 2 bis 10 mm insbesondere 2 bis 5 mm.

In einer weiteren bevorzugten Ausführungsform der vorliegenden Erfindung umfasst die Trägerplatteneinheit ein Reservoir für Flüssigkeiten, insbesondere für Zellkulturmedium oder zu untersuchende Wirkstoffe.

Bevorzugt umfasst die Trägerplatteneinheit neben der ersten Trägerplatte, bevorzugt neben der ersten und zweiten Trägerplatte, ein Reservoir für Flüssigkeiten, insbesondere für Zellkulturmedium oder zu untersuchende Wirkstoffe.

Bevorzugt umfasst die Trägerplatteneinheit eine erste Trägerplatte, eine bevorzugt über der ersten Trägerplatte angeordnete zweite Trägerplatte und ein bevorzugt über der zweiten Trägerplatte angeordnetes Reservoir für Flüssigkeiten, insbesondere für Zellkulturmedium oder zu untersuchende Wirkstoffe.

In besonders bevorzugter Ausführungsform der vorliegenden Erfindung weist das Reservoir mindestens ein separates proximal zur zentral gelegenen Rotationsachse der Trägerplatteneinheit angeordnetes Behältnis, bevorzugt Medienbehältnis, auf.

In einer weiteren bevorzugten Ausführungsform der vorliegenden Erfindung weist das Reservoir mindestens ein separates proximal zur zentral gelegenen Rotationsachse der Trägerplatteneinheit angeordnetes Behältnis, bevorzugt Medienbehältnis, und mindestens ein separates distal zur zentral gelegenen Rotationsachse der Trägerplatteneinheit angeordnetes Behältnis, bevorzugt Medienbehältnis auf, wobei das proximal zur zentral gelegenen Rotationsachse der Trägerplatteneinheit angeordnete Behältnis, bevorzugt Medienbehältnis, und das distal zur zentral gelegenen Rotationsachse der Trägerplatteneinheit angeordnete Behältnis, bevorzugt Medienbehältnis, in Fluidverbindung miteinander stehen.

Bevorzugt umfasst das mindestens eine separate proximal zur zentral gelegenen Rotationsachse der Trägerplatteneinheit angeordnete Behältnis, bevorzugt Medienbehältnis, mindestens eine Medienöffnung, bevorzugt mindestens einen Medienauslass.

Bevorzugt umfasst das mindestens eine separate distal zur zentral gelegenen Rotationsachse der Trägerplatteneinheit angeordnete Behältnis, bevorzugt Medienbehältnis, mindestens eine Medienöffnung, bevorzugt mindestens einen Medieneinlass.

Gemäß einer bevorzugten Ausführungsform wird eine in dem mindestens einen separaten proximal zur zentral gelegenen Rotationsachse der Trägerplatteneinheit angeordneten Behältnis, bevorzugt Medienbehältnis, befindliche Flüssigkeit, bevorzugt Zellkulturmedium, bei Rotation der Trägerplatteneinheit um die zentrale Rotationsachse durch die mindestens eine Medienöffnung, bevorzugt durch den mindestens einen Medienauslass, des proximal zur zentral gelegenen Rotationsachse der Trägerplatteneinheit angeordneten Behältnisses der fluidisch verbundenen Medienöffnung, bevorzugt dem fluidisch verbunden Medieneinlass, der zweiten Trägerplatte zugeführt und über mindestens einen in der zweiten Trägerplatteneinheit befindlichen unverzweigten oder verzweigten Medienkanal zur mindestens einen Medienöffnung, bevorzugt zum mindestens einen Mcdicnauslass der zweiten Trägcrplattc befördert. Von der mindestens einen Medienöffnung, bevorzugt dem mindestens einen Medienauslass der zweiten Trägerplatte wird die Flüssigkeit, bevorzugt das Zellkulturmedium, über die mindestens eine fluidisch verbundene Medienöffnung, bevorzugt den mindestens einen fluidisch verbundenen Medieneinlass, des mindestens einen separaten distal zur zentral gelegenen Rotationsachse der Trägerplatteneinheit angeordneten Behältnisses, bevorzugt Medienbehältnisses, zugeführt. Durch die bevorzugt kontinuierliche oder pulsative Strömung von Flüssigkeit, bevorzugt Zellkulturmedium, vom proximal zur zentral gelegenen Rotationsachse der Trägerplatteneinheit angeordneten Behältnis, bevorzugt Medienbehältnis, zum distal zur zentral gelegenen Rotationsachse der Trägerplatteneinheit angeordneten Behältnis, bevorzugt Medienbehältnis, über den Medienkanal und die Medienkammern der zweiten Trägerplatte kann eine Versorgung der in der mindestens einen Kultivierungskammer der ersten Trägerplatte befindlichen Zellen mit Flüssigkeit, bevorzugt Zellkulturmedium, gewährleistet werden.

Auf diese Weise ist es vorteilhafterweise möglich, in der mindestens einen Kultivierungskammer der erfindungsgemäßen Vorrichtung befindliche Zellen, bevorzugt kontinuierlich oder pulsativ, mit größeren Mengen an Flüssigkeit, bevorzugt Zellkulturmedium, zu versorgen.

In bevorzugter Ausführungsform ist die Trägerplatteneinheit, insbesondere die erste und/oder zweite Trägerplatte, bevorzugt die erste und/oder zweite Trägerplatte und/oder das Reservoir, aus Glas oder einem polymeren Material aufgebaut. Besonders bevorzugt ist die Trägerplatteneinheit aus einem polymeren Material, beispielsweise PDMS (Polydimethylsiloxan), PMMA (Polymethylmethylacrylat), PVC (Polyvinylchlorid), COC (Cycloolefin Copolymere), PS (Polystyren), PC (Polycarbonat), Polyimid, Polyurethan, PET (Polyethylenterephthalat), Polyester, insbesondere Polycaprolacton (PCT), oder Kombinationen davon aufgebaut.

Die vorliegende Erfindung betrifft weiterhin ein Verfahren zum Kultivieren von Zellen, wobei die Zellen in einer erfindungsgemäßen Vorrichtung kultiviert werden. Hierbei handelt es sich bevorzugt um menschliche oder tierische Zellen, insbesondere Zellsuspensionen solcher Zellen.

Insbesondere erfolgt die Kultivierung der Zellen in der erfindungsgemäßen Vorrichtung durch: a) Bereitstellen der Zellen, gegebenenfalls in Form von Suspensionen, zum Beispiel in Zellkulturmedium, vernetztem oder unvernetztem Hydrogel, und einer erfindungsgemäßen Vorrichtung, b) Einbringen der Zellen, gegebenenfalls in Form von Suspensionen, zum Beispiel in Zcllkulturmcdium, vernetztem oder unvernetztem Hydrogel, in die erfindungsgemäße Vorrichtung durch die mindestens eine Zugangsöffnung, c) Einbringen der erfindungsgemäßen Vorrichtung in eine eine Rotation ermöglichende Drehvorrichtung, d) in Rotation versetzen der erfindungsgemäßen Vorrichtung, e) Erhalt von Zellen in der mindestens einen Kultivierungskammer und f) Kultivieren der Zellen in der mindestens einen Kultivierungskammer. Es kann erfindungsgemäß auch vorgesehen sein, dass Schritt c) vor Schritt b) durchgeführt wird. Besonders bevorzugt entsteht durch das Kultivieren der Zellen in der mindestens einen Kultivierungskammer ein Zellverband, Gewebeverband oder eine Zellkultur mit besonders hoher Zelldichte.

In besonders bevorzugter Ausführungsform wird die erfindungsgemäße Vorrichtung vor der Verwendung zunächst in O₂-Plasma aktiviert, um bei Befüllung eine bessere Benetzung der Kanäle und/oder Medienkanäle zu erreichen. Als Parameter werden insbesondere 500 W, 60 s und 1 mbar verwendet.

Im erfindungsgemäßen Verfahren zur Kultivierung von Zellen kann es bevorzugt auch vorgesehen sein, dass vor Durchführung von Verfahrensschritt b) in einem Verfahrensschritt x) ein Einbringen von Zellkulturmedium durch die mindestens eine Zugangsöffnung vorgesehen ist, welches anschließend in einem Verfahrensschritt y) durch Rotation, das heißt die Zentrifugalkraft, durch den mindestens einen die mindestens eine Zugangsöffnung mit der mindestens einen Kultivierungskammer verbindenden Kanal in die Kultivierungskammer transportiert wird. Dies bewirkt ein Entfernen von Luftblasen, die bei der Kultivierung der Zellen störend wirken. Anschließend ist erfindungsgemäß bevorzugt vorgesehen, die Verfahrensschritte b) bis d), insbesondere b) bis f) durchzuführen. Erfindungsgemäß bevorzugt werden weiterhin die Verfahrensschritte g) Einbringen von Zellkulturmedium in die mindestens eine Medienöffnung, h) in Rotation versetzen der Vorrichtung und i) Erhalt von Zellkulturmedium in der mindestens einen Medienkammer zur Versorgung der Zellen in der Kultivierungskammer durchgeführt.

In einer weiteren bevorzugten Ausführungsform der vorliegenden Erfindung umfasst das Verfahren zur Kultivierung von Zellen in Verfahrensschritt g) das Einbringen von Zellkulturmedium in das mindestens eine separate proximal zur zentral gelegenen Rotationsachse der Trägerplatteneinheit angeordnete Behältnis, bevorzugt Medienbehältnis, des Reservoirs, h) in Rotation Versetzen der Vorrichtung und i) Erhalt von Zellkulturmedium in der mindestens einen Medienkammer zur Versorgung der Zellen in der Kultivierungskammer.

Insbesondere wird dabei ein kontinuierliches oder pulsatives Durchströmen der Medienkanäle und Medienkammern mit Zellkulturmedium durch die Erzeugung eines Druckgradienten, externen oder integrierten Pumpen oder durch Rotation der Vorrichtung ermöglicht.

Das für die Kultivierung der Zellen verwendete Zellkulturmedium ist bevorzugt für die verwendete Zellspezies angepasst, beispielsweise wird für Fibroblasten DMEM mit 10 % FBS und 1 % Penicillin / Streptomycin verwendet. Gegebenenfalls kann das Zellkulturmedium Biomoleküle zur Zellanhaftung enthalten. Insbesondere wird in Schritt x) Zellkulturmedium in die Zugangsöffnungen pipettiert, wobei das Volumen je nach Vorrichtungsgeometrie an das Kultivierungskammervolumen angepasst wird. Bevorzugt wird ein Volumen von 1-40 µl, insbesondere 10 - 20 µl Zellkulturmedium in die Zugangsöffnung(en) eingebracht und anschießend in Verfahrensschritt y) durch Rotation in die Kultivierungskammern transportiert. In Verfahrensschritt b) werden die Zellen, insbesondere die im Zellkulturmedium oder Hydrogel vorliegenden Zellen in die Zugangsöffnungen eingebracht, wobei die Zellkonzentration dem Kultivierungskammervolumen und gewünschten Füllanteil, sowie dem verwendeten Zelltyp und der spezifischeren Zellgeometrie angepasst ist. Erfindungsgemäß bevorzugt werden 1-40 µl, insbesondere 10 - 20 µl Zellkulturmedium oder Hydrogel mit einer Zellkonzentration von 10³ bis 10⁸, insbesondere 10⁴ - 10⁷ / 10 µl in die Zugangsöffnung eingebracht.

In bevorzugter Ausführungsform erfolgt das Einbringen von Zellkulturmedium, Hydrogel und Zellen durch Pipettieren, Injizieren oder andere geeignete Verfahren.

In besonders bevorzugter Ausführungsform kann vorgesehen sein, nach Verfahrensschritt e) über die mindestens eine Medienöffnung die mindestens eine Medienkammer und damit auch die mit der mindestens einen Medienkammer fluidisch verbundene mindestens eine Kultivierungskammer mit Medium zu versorgen und eine besonders bevorzugte Kultivierung durchzuführen. Dieser Verfahrensschritt kann bevorzugt nach Beendigung der Rotation durchgeführt werden, sodass insbesondere bevorzugt vorgesehen ist, eine Mediumsversorgung und Kultivierung der Zellen im Stillstand der erfindungsgemäßen Vorrichtung durchzuführen. Weiterhin kann es erfindungsgemäß auch vorgesehen sein, dass die Rotation zur Mediumsversorgung ausgenutzt wird und so die Kultivierung der Zellen, das heißt Schritt f) des erfindungsgemäßen Verfahrens, in Rotation stattfindet.

In einer bevorzugten Ausführungsform der vorliegenden Erfindung kann vorgesehen sein nach Verfahrensschritt e) über die mindestens eine Medienöffnung, bevorzugt den mindestens einen Medienauslass, des mindestens einen separaten proximal zur zentral gelegenen Rotationsachse der Trägerplatteneinheit angeordneten Behältnisses, bevorzugt Medienbehältnisses, des Reservoirs und die damit fluidisch verbundene mindestens eine Medienöffnung der zweiten Trägerplatte die mindestens eine Medienkammer und damit auch die mit der mindestens einen Medienkammer fluidisch verbundene mindestens eine Kultivierungskammer mit Medium zu versorgen und eine besonders bevorzugte Kultivierung durchzuführen. Gemäß dieser Ausführungsform ist vorgesehen, dass die Rotation zur Mediumsversorgung ausgenutzt wird und so die Kultivierung der Zellen, das heißt Schritt f) des erfindungsgemäßen Verfahrens, in Rotation stattfindet.

In besonders bevorzugter Ausführungsform erfolgt die Rotation der erfindungsgemäßen Vorrichtung mit einer Umdrehungsgeschwindigkeit von 0 bis 4000 rpm, insbesondere für 0,1 bis 30 Minuten, insbesondere mit 1500 bis 2500 rpm, insbesondere für 60 bis 360 Sekunden. Diese Parameter gelten insbesondere für die Befüllung der Kultivierungskammern, Medienkammern, Kanäle und Medienkanäle. Sofern eine Kultivierung der Zellen unter Rotation vorgesehen ist, das heißt die Rotation zur Mediumsversorgung der Zellen ausgenutzt wird, so ist bevorzugt eine Umdrehungsgeschwindigkeit von 0 bis 1000 rpm, insbesondere mit 0 bis 100 rpm, bevorzugt mit 10 bis 50 rpm vorgesehen.

Erfindungsgemäß bevorzugt entsteht durch das Kultivieren der Zellen in der mindestens einen Kultivierungskammer der erfindungsgemäßen Vorrichtung ein Zellverband.

Die vorliegende Erfindung betrifft auch ein Verfahren zur Herstellung eines Zellverbandes, insbesondere eines dreidimensionalen Zellverbandes, dadurch gekennzeichnet, dass das erfindungsgemäße Verfahren zum Kultivieren von Zellen durchgeführt und ein Zellverband erhalten wird.

Die vorliegende Erfindung betrifft auch ein Verfahren zur Herstellung einer Vorrichtung gemäß der vorliegenden Erfindung, insbesondere ist ein derartiges Verfahren ein Verfahren im Rahmen dessen in einem ersten Verfahrensschritt mindestens ein die Trägerplatteneinheit bildendes Material, insbesondere polymeres Material, bereitgestellt und dieses in einem Form- und Stabilität gebenden Verfahren, insbesondere einem lithographischen Verfahren, bevorzugt einem Soft- oder UV-lithographischen Verfahren, zu einer Vorrichtung gemäß der vorliegenden Erfindung ausgestaltet wird.

Das heißt, bevorzugt wird die erfindungsgemäße Vorrichtung mithilfe von Photolithographie, Softlithographie, selektivem Lasersintern, Laser-Schneiden und Fräsen, Laserablation, Tintenstrahldruck mit Photopolymeren, Schmelzbeschichtung (Thermoplastische Extrusion), LOM (Laminated Object Manufacturing), Stereolithographie, Heißprägen (Hot Embossing, insbesondere Mikroheißprägen), Fräsen (CNC-Fräsen, insbesondere Mikrofräsen), Kunststoffspritzguss (Injection moulding, insbesondere Mikrospritzguss) und/oder 3D-Druck hergestellt. Diese Verfahren können entweder direkt zur Herstellung der Vorrichtung oder zur Herstellung von Formvorlagen und anschließendem Abformen der Vorrichtung verwendet werden.

Insbesondere betrifft die vorliegende Erfindung auch ein Verfahren zur Herstellung einer Vorrichtung gemäß der vorliegenden Erfindung, umfassend die Verfahrensschritte
i) Bereitstellen mindestens eines Negativ-Resist-Materials, mindestens eines Silizium-Substrats und mindestens einem die Trägerplatteneinheit bildenden Material,
ii) Herstellen einer Formvorlage für eine Trägerplatteneinheit, insbesondere einer ersten Trägerplatte, aus dem Negativ-Resist-Material auf dem Silizium-Substrat durch UV-Lithographie,
iii) Ausgießen der Formvorlage mit dem die Trägerplatteneinheit bildenden Material,
iv) Aushärten des die Trägerplatteneinheit bildenden Materials in der Formvorlage und
v) Erhalten der Trägerplatteneinheit, insbesondere einer ersten Trägerplatte.

Die vorliegende Erfindung betrifft auch ein vorstehendes erfindungsgemäßes Verfahren zur Herstellung einer Vorrichtung, wobei zusätzliche in Verfahrensschritt ii) das Herstellen einer Formvorlage für eine zweite Trägerplatte, wobei zusätzlich in Verfahrensschritt iii) das Ausgießen der Formvorlage für die zweite Trägerplatte mit dem die Trägerplatteneinheit bildenden Material, zusätzlich in Verfahrensschritt iv) das Aushärten des die Trägerplatteneinheit bildenden Materials in der Formvorlage für die zweite Trägerplatte und zusätzlich im Verfahrensschritt v) das Erhalten einer zweiten Trägerplatte und das anschließende Zusammenfügen mit der ersten Trägcrplattc umfasst ist.

Besonders bevorzugt wird als Trägerplatteneinheit bildendes Material PDMS verwendet, in Schritt iii) in die lithographisch hergestellte Form gegossen und für 0.5 - 12 h, bevorzugt 1 - 2 h bei 50 - 100 °C, bevorzugt 60 - 80 °C ausgehärtet.

In einer weiteren bevorzugten Ausführungsform ist vorgesehen, dass zusätzlich in Verfahrensschritt i) das Bereitstellen mindestens einer Trennvorrichtung, insbesondere mindestens einer Membran und zusätzlich in Verfahrensschritt v) das Zusammenfügen der ersten Trägerplatte, der mindestens einen Trennvorrichtung und der zweiten Trägerplatte vorgesehen ist.

Sofern lithographisch hergestellten Membranen (beispielsweise auf der Basis von 1002F-50 oder SU-8) verwendet werden, werden zum Zusammenfügen der Vorrichtung die erste Trägerplatte, die Membran und die zweite Trägerplatte, mit Hilfe von N₂-Plasma miteinander verbunden. Für andere Membranmaterialien ist auch ein Verbinden mit Hilfe von O₂-Plasma möglich.

Insbesondere wird zum Zusammenfügen zunächst je nach Membrangeometrie entweder die erste oder die zweiter Trägerplatte in N₂-Plasma aktiviert, wofür bevorzugt Parameter von 30 - 100 W, 30 - 120 s und 0.3 - 1.5 mbar verwendet werden. Die aktivierte Trägerplatte wird auf der sich auf dem Silizium-Substrat befindlichen Membran ausgerichtet, mit Gewicht beschwert und die Bindung bei 60 - 120 °C für 0.5 - 24 h im Ofen ausgehärtet. Anschließend wird die kombinierte Schicht aus Trägerplatte und Membran in H₂O vom Si-Substrat abgelöst. Die weitere Trägerplatte wird nun mit den oben genannten Parametern in einem N₂-Plasma aktiviert, auf der freien Membranseite ausgerichtet, beschwert und die Bindung bei oben genannten Parametern im Ofen ausgehärtet. Zur Befestigung am Motor werden in der zusammengefügten und ausgehärteten Disk zusätzlich 4 Durchgangsöffnungen in der Mitte erstellt.

Weitere vorteilhafte Ausgestaltungen der vorliegenden Erfindungen ergeben sich aus den Unteransprüchen.

Die Erfindung wird anhand des folgenden Beispiels und der zugehörigen Figuren näher erläutert.

Die Figuren zeigen:
Figur 1 zeigt schematisch eine erfindungsgemäße Trägerplatteneinheit (110).
Figur 2 zeigt schematisch den Aufbau einer ersten Trägerplatte (111).
Figur 3 zeigt einen Ausschnitt der ersten Trägerplatte (111).
Figur 4 zeigt die überlappend angeordneten Kultivierungskammern (140) der ersten Trägerplatte (111) und Medienkammern (170) der zweiten Trägerplatte (112).
Figur 5 zeigt einen Querschnitt durch die erfindungsgemäße Vorrichtung.
Figur 6 zeigt schematisch den Aufbau einer ersten Trägerplatte (111).
Figur 7 zeigt eine erste Trägerplatte (111) mit eingefärbten Kultivierungskammern (140) und Kanälen (150).
Figur 8 zeigt mögliche Kultivierungskammergeometrien a) rund, b) rechteckig und c) hantelförmig.
Figur 9 zeigt schematisch den Aufbau einer zweiten Trägerplatte (112).
Figur 10 zeigt schematisch den Aufbau der Membran (135).
Figur 11 zeigt eine erfindungsgemäße Vorrichtung mit erster und zweiter Trägerplatte (111, 112) und dazwischenliegender Membran (135).
Figur 12 zeigt die notwendigen Schritte zum Befüllen der Kultivierungskammern (140).
Figur 13 zeigt eine mit Zellen befüllte runde Kultivierungskammer (140).
Figur 14 zeigt eine mit Zellen befüllte hantelförmige Kultivierungskammer (140).
Figur 15 zeigt eine mit Zellen befüllte lasergeschnittene Kultivierungskammer (140).
Figur 16 zeigt einen um den Winkel α geneigten Kanal (150) mit Kultivierungskammern (140).
Figur 17 zeigt schematisch die durch Rotation bewirkte Befüllung der Kultivierungskammern.
Figur 18 zeigt acht mit Zellen befüllte Kultivierungskammern (140).
Figur 19 zeigt schematisch einen möglichen Aufbau der ersten Trägerplatte (111).
Figur 20 zeigt schematisch einen weiteren möglichen Aufbau der ersten Trägerplatte (111).
Figur 21 zeigt schematisch eine Ausführungsform, bei der die Zugangsöffnung (130) als Ladekammer (190) ausgebildet ist.
Figur 22 zeigt schematisch die erfindungsgemäße Vorrichtung in Mikrotiter-Format.
Figur 23 zeigt den Aufbau eines Reservoirs (200) für Zellkulturmedium oder zu untersuchende Wirkstoffe.
Figur 24 zeigt den experimentell bestimmten und berechneten Volumenstrom in einer erfindungsgemäßen Vorrichtung in Abhängigkeit von der Rotationsgeschwindigkeit.

### Beispiel

### Herstellung einer erfindungsgemäßen Vorrichtung

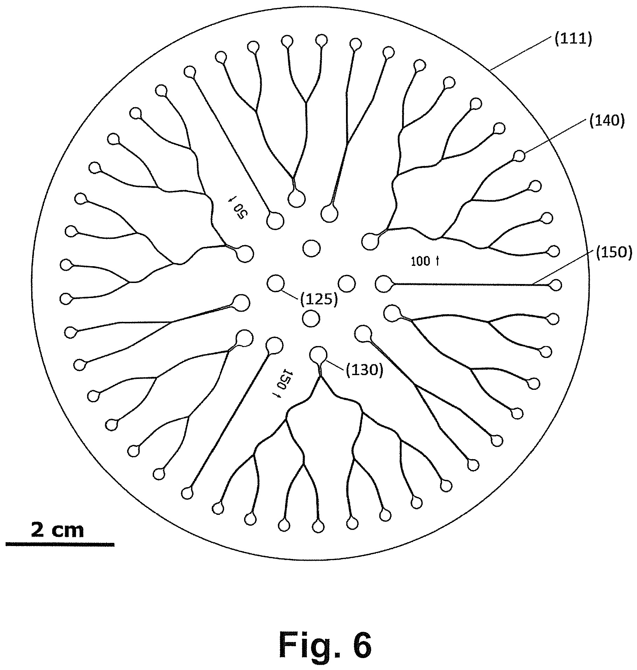

Im Folgenden wird die Herstellung einer beispielhaften Organ-Disk beschrieben. Die erfindungsgemäße Disk, das heißt die Trägerplatteneinheit, wird aus Polydimethylsiloxan (PDMS, bezogen von Dow Corning als Sylgard 184) hergestellt. Die einzelnen Trägerplatten werden durch Soft-Lithographie hergestellt wobei zunächst mittels UV-Lithographie aus dem Photolack SU-8 eine Formvorlage der jeweiligen Trägerplatte in der benötigten Kanalhöhe auf Silizium-Substrat (Wafer) angefertigt wird. Die Formvorlagen weisen folgende Eigenschaften für die beschriebene Disk auf:
Die erste Trägerplatte der beispielhaften Disk enthält 45 im radialen Abstand von 4.5 cm zum Diskmittelpunkt, das heißt der zentralen Rotationsachse, angeordnete Kultivierungskammern mit einem Durchmesser von 2 mm. Figur 6 zeigt das verwendete Trägerplattendesign. In der Mitte der Trägerplatteneinheit sind zusätzlich 4 Durchgangsöffnungen zur Befestigung der Disk am Motor angebracht. Die Kammern werden über 12 Zugangsöffnungen durch Kanäle der Höhe von 50 µm gefüllt. Jeweils 1/3 der die Zugangsöffnung mit der Kultivierungskammer verbindenden Kanäle weist eine Kanalbreite von 50 µm, 100 µm, sowie 150 µm auf. Pro Kanalbreite gibt es 4 verschiedene Arten von symmetrischen Verzweigungen: 0 Verzweigungen (Zugangsöffnung direkt mit der Kammer verbunden), 1 Verzweigung (Zugangsöffnung mit 2 Kammern verbunden), 2 Verzweigungen (Zugangsöffnung mit 4 Kammern verbunden), 3 Verzweigungen (Zugangsöffnung mit 8 Kammern verbunden). Der Zugangsöffnungsdurchmesser sowie der Durchmesser der Durchgangsöffnungen betragen 3 mm.

Die zweite Trägerplatte beinhaltet Medienkammern welche passend zu den Kultivierungskammern der ersten Trägerplatte angeordnet sind. Pro Medienöffnung gibt es einen Medienkanal welcher alle mit der Medienöffnung verbundenen Medienkammern versorgt. Alle Medienkanäle weisen eine Breite von 80 µm sowie eine Höhe von 50 µm auf. Jeder Medienkanal besitzt zwei Medienöffnungen, wobei eine Medienöffnung als Medienauslass fungiert und außerhalb des Kanals der ersten Trägerplatte liegt. Die Medienöffnungen besitzen einen Medienöffnungsdurchmesser von 1 mm.

PDMS wird zunächst im Verhältnis von 10:1 aus den beiden Komponenten Base:Agent angemischt und im Exsikkator für 30 Minuten unter Vakuum entgast. 21 g PDMS werden jeweils in die lithographisch hergestellte Formvorlage für die erste sowie zweite Trägerplatte gegossen und für 14 h bei 60 °C ausgehärtet. Das verwendete Silizium-Substrat mit den abzuformenden Strukturen besitzt einen Durchmesser von 10 cm. Durch einen zusätzlichen Acrylring welcher auf das Silizium-Substrat geklemmt ist wird ein Abfließen des PDMS vermieden und die Disk zu einem finalen Diskdurchmcsscr von 9,5 cm geformt. Um Unregelmäßigkeiten in der Trägerplattendicke im Randbereich der Disk zu vermeiden wird der Acrylring bis oben hin mit PDMS gefüllt, was zu einer Höhe der ersten sowie zweiten Trägerplatte von 3 mm führt.

Nach dem Aushärten der beiden Trägerplatten werden die in der ersten sowie zweiten Trägerplatte vorgeformten Durchgangslöcher ausgestanzt. In der zweiten Trägerplatte werden zusätzlich Zugangsöffnungen mit einem Durchmesser von 3 mm sowie Medienöffnungen mit einem Durchmesser von 1 mm ausgestanzt. Beide Trägerplatten werden nun mit Isopropanol abgespült und mit Stickstoff getrocknet. Die Trägerplatten werden zusätzlich mit Klebeband bedeckt welches wieder abgezogen wird um Verunreinigungen auf der Oberfläche zu entfernen.

Für das dargestellte Beispiel wird eine Membran auf Basis eines Epoxid-Photolacks (1002F) verwendet. Die Membran wird in Trägerplattengröße für die ganze Disk hergestellt. Das Design der verwendeten Membran für die komplette Disk ist in Figur 10 gezeigt. Hierbei ist die Diskmembran in 4 Quadranten mit den Porengrößen von 3 µm (I), 3 µm (II), 5 µm (III), 3 µm (IV) und den Porositäten 12.7 % (I), 5.6 % (II), 5.6 % (III), 3.2 % (IV) eingeteilt. Die durchlässigen Poren sitzen in einem hexagonalen Gitter angeordnet nur im Randbereich, mit radialem Abstand zwischen 4.0 und 4.6 cm, das heißt am Überlapp zwischen den Kultivierungskammern und den Medienkammern um die Diffusion in diesem Bereich zu gewährleisten. Zusätzlich ist die Membran an Stelle der Zugangsöffnungen durchlässig. Die etwa 10 µm dicke Membran befindet sich nach der Herstellung, mittels Lithographie im Reinraum auf einem Silizium-Wafer und kann in H₂O abgelöst werden.

Zum Zusammenbau der Organdisk werden die einzelnen Schichten mit Hilfe von N₂-Plasma aneinander gebondet, das heißt miteinander verbunden. Dafür wird zunächst die erste Trägerplatte in N₂-Plasma aktiviert, wofür Parameter von 50 W, 90 s und ein Fluss von 0.2 Nl/h verwendet werden. Die aktivierte Trägerplatte wird auf der sich auf dem Silizium-Substrat befindlichen Membran ausgerichtet, mit Gewicht beschwert und die Bindung bei 60 °C über Nacht, d.h. für mindestens 14 h im Ofen ausgehärtet. Anschließend wird die kombinierte Schicht aus Trägerplatte und Membran in H₂O (Milli-Q Reinstwasser) eingelegt. Nach etwa 5 Minuten löst sich die Seifenschicht unter der Membran, so dass die kombinierte Schicht vom Silizium-Substrat abgelöst werden kann. Die zweite Trägerplatte wird nun mit den oben genannten Parametern ebenfalls in einem N₂-Plasma aktiviert, auf der freien Membranseite ausgerichtet, beschwert und die Bindung bei oben genannten Parametern im Ofen ausgehärtet. Nach diesem Schritt ist die Disk fertiggestellt und einsatzbereit.

### Anwendung der Disk

In dem vorliegenden Beispiel wird die Disk dazu verwendet, Fibroblasten in den Kultivierungskammern anzureichern und diese später zu kultivieren. Zur Anwendung der Disk wird diese zunächst in O₂-Plasma aktiviert, um bei Befüllung eine bessere Benetzung der Kanäle zu erreichen. Als Parameter werden 50 W, 60 s und ein O₂₋Fluss von 0.2 Nl/h verwendet.

Es werden 10 µl des Zellkulturmediums für Fibroblasten (DMEM mit 10 % FBS und 1 % Penicillin / Streptomycin) in die Zugangsöffnungen pipettiert. Anschließend wird die Organdisk mit einem Deckel abgeschlossen und über die Durchgangslöcher auf den Motor geschraubt.

Die Disk wird für 3 Min mit 2000 rpm in Rotation gebracht, so dass die Kanäle sowie Kultivierungskammern der ersten Trägerplatte mit Medium gefüllt werden.

Anschließend wird die Disk vom Motor wieder entfernt, der Deckel geöffnet und das restliche Mediums-Volumen in den Zugangsöffnungen mit einer Pipette abgesaugt. Im nächsten Schritt wird die Zellsuspension in die Zugangsöffnungen pipettiert. Hierfür werden im Beispiel 10 µl von einer Zellsuspension Fibroblasten mit einer Konzentration von 10⁵ Zellen / 10 µl verwendet.

Die Disk wird wieder verschlossen und am Motor angebracht. Die anschließende Diskrotation mit 2000 rpm für 3 Min befördert die eingefüllten Zellen in die Kultivierungskammern. Dies entspricht einer, zur Zentrifugation von Fibroblasten üblichen, Beschleunigung von 200 g (g für den äußeren Rand der Kammern bei r₂= 0,045 m: a= 5 ^{∗} 10^{-5 ∗} rpm²g bei einer Rotationsgeschwindigkeit von ω = 2000 rpm).

Jetzt sind die Kultivierungskammern mit Fibroblasten befüllt, so dass die Disk vom Motor entfernt und im Stillstand verwendet werden kann. Dazu wird über die Medienöffnungen ein definierter Mediumsstrom mit Hilfe externer Pumpen bereitgestellt.

### Herstellung eines Zellverbandes

Zur Herstellung eines Zellverbandes mittels des erfindungsgemäßen Verfahrens wurde eine Vorrichtung mit einem gegenüber der Richtung der durch Rotation erzeugten Zentrifugalkraft F_{C} geneigten Kanals (150) eingesetzt, entlang dessen Länge acht Kultivierungskammern (140) angeordnet sind.

Zur Entlüftung des Kanals (150) wurde die Vorrichtung zunächst für die Dauer von 2 Minuten bei 200 g rotiert. Anschließend wurde eine 80.000 Kardiomyozyten enthaltende Suspension in die Zugangsöffnung (130) gegeben. Nach Verschließen der Zugangsöffnung (130) wurde die Vorrichtung für 10 Minuten bei 200 g zentrifugiert, wodurch die Kardiomyozyten in die Kultivierungskammern (140) befördert wurden.

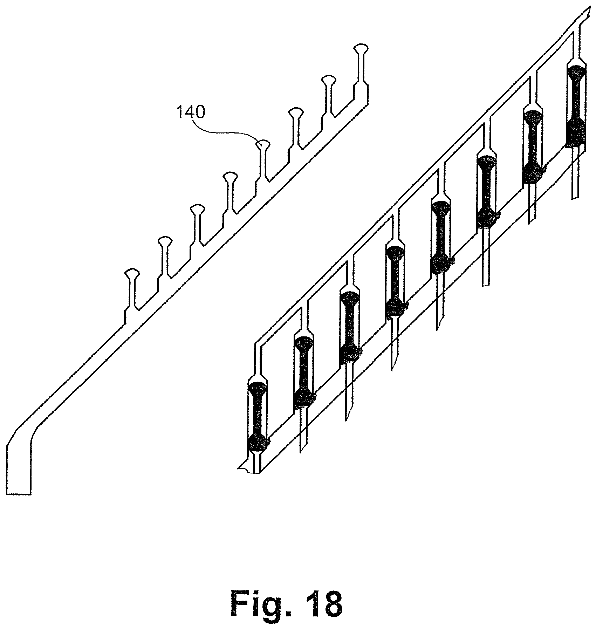

Figur 18 zeigt die Bildung eines dichten dreidimensionalen Zellverbands in den acht Kultivierungskammern (140) der Vorrichtung. Zur weiteren Kultivierung des erhaltenen Zellverbandes wurden die Zellen in den Kultivierungskammern (140) anschließend durch eine externe Spritzenpumpe bei einer Flussrate von 50µl/h mit Medium versorgt.

### Messung des durch Rotation geförderten Volumenstroms

Eine Messung des durch Rotation geförderten Volumenstroms erfolgte durch gravimetrische Messung des aufgefangenen Fördervolumens. Dazu wurde der Volumenstrom von Wasser aus einem Reservoir mit konstantem Füllstand durch einen Medienkanal einer erfindungsgemäßen Vorrichtung bei unterschiedlichen Rotationsgeschwindigkeiten (rpm) bestimmt (Tabelle 1).

**Tabelle 1: Messwerte der gravimetrischen Flussmessung und theoretisch berechnete Flussraten.**

| Geschwindigkeit [rpm] | Theo. Fluss [µL / h] | Mittlerer Fluss [µL / h] | Standardabweichung [µL / h] | Anzahl Messwerte [-] |
|---|---|---|---|---|
| 100 | 64 | 86 | 7 | 12 |
| 200 | 144 | 118 | 31 | 12 |
| 300 | 277 | 214 | 35 | 9 |
| 400 | 463 | 414 | 65 | 12 |
| 500 | 702 | 715 | 69 | 12 |
| 600 | 994 | 1011 | 89 | 12 |
| 700 | 1339 | 1457 | 99 | 8 |
| 800 | 1737 | 1783 | 163 | 11 |

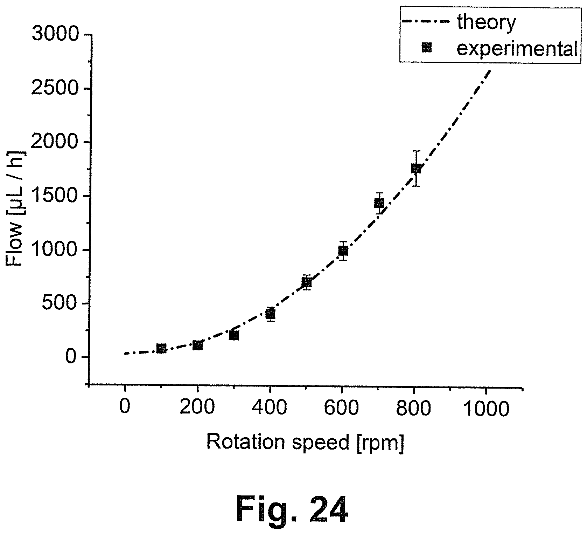

Figur 24 veranschaulicht, dass der durch gravimetrische Messung in Abhängigkeit von der Rotationsgeschwindigkeit bestimmte Volumenstrom von Wasser in einer erfindungsgemäßen Vorrichtung mit den theoretisch berechneten Volumenströmen nahezu übereinstimmt.

Der Versuch zeigt somit, dass es mit der erfindungsgemäßen Vorrichtung möglich ist, auch ohne externe Pumpen Flüssigkeiten, insbesondere Medium, durch Rotation um eine zentrale Rotationsachse von der mindestens einen proximal zur zentralen Rotationsachse angeordneten Zugangsöffnung über den Kanal zu der mindestens einen distal zur zentralen Rotationsachse angeordneten Kultivierungskammer zu befördern.

### Figurenbeschreibung

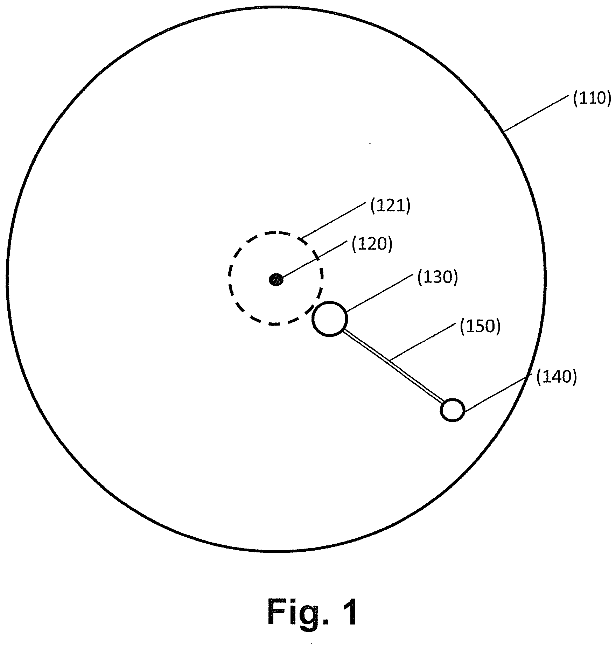

Figur 1 zeigt eine erfindungsgemäße Trägerplatteneinheit (110) in Form einer Scheibe. Trägerplatteneinheit (110) weist eine zentrale Rotationsachse (120) und einen diese umgebenden Zentralbereich (121) auf. Proximal zur zentralen Rotationsachse (120) ist eine Zugangsöffnung (130) angeordnet, die über Kanal (150) mit Kultivierungskammer (140) verbunden ist.

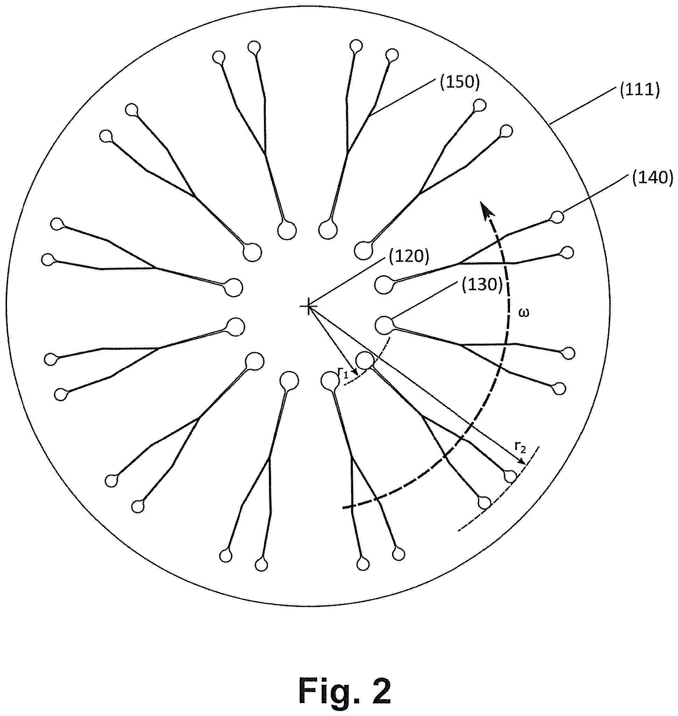

Figur 2 zeigt eine erste Trägerplatte (111) mit zentraler Rotationsachse (120) und proximal dazu in Abstand r₁ angeordneten Zugangsöffnungen (130) und distal in Abstand r₂ zur zentralen Rotationsachse (120) angeordneten Kultivierungskammern (140), wobei der die Zugangsöffnungen (130) mit den Kultivierungskammern (140) verbindende Kanal (150) ein verzweigter Kanal ist.

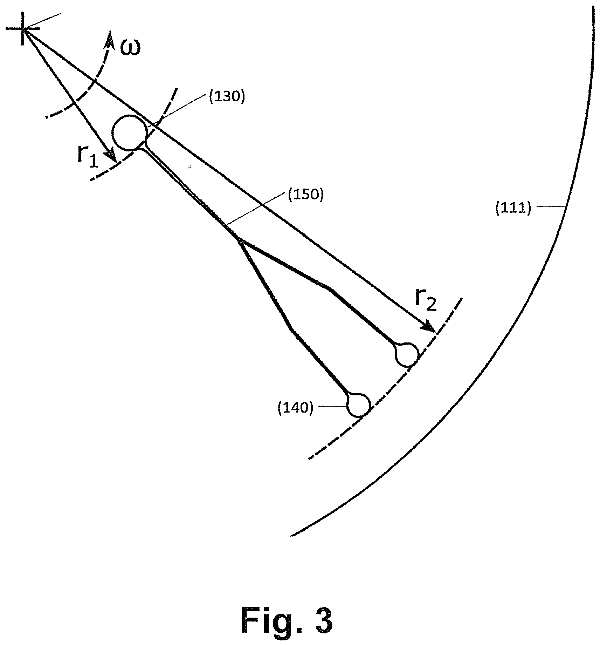

Figur 3 zeigt ausschnittsartig die zentrale Rotationsachse (120) der ersten Trägerplatte (111) sowie die proximal zur zentralen Rotationsachse (120) in Abstand r₁ angeordnete Zugangsöffnung (130), die distal in Abstand r₂ angeordnete Kultivierungskammer (140) und den die Zugangsöffnung (130) und die Kultivierungskammern (140) verbindenden verzweigten Kanal (150). Durch Rotation der Trägerplatte mit Winkelgeschwindigkeit ω werden die Kultivierungskammern (140) mit Zellen befüllt.

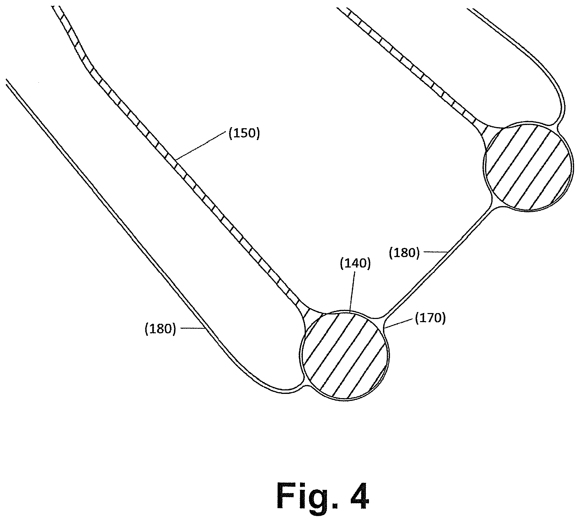

Figur 4 zeigt zwei Kultivierungskammern (140) der ersten Trägerplatte, welche überlappend mit zwei Medienkammern (170) der zweiten Trägerplatte angeordnet und durch eine Membran getrennt sind. Kanal (150) verbindet (nicht gezeigte) Zugangsöffnungen mit den Kultivierungskammern (140) der ersten Trägerplatte und der Medienkanal (180) verbindet die (nicht gezeigten) Medienöffnungen mit einer ersten Medienkammer (170) sowie einer zweiten Medienkammer (170) der zweiten Trägerplatte. Die Kultivierungskammern (140) und Kanäle (150) der ersten Trägerplatte sind mit Strichen dargestellt.

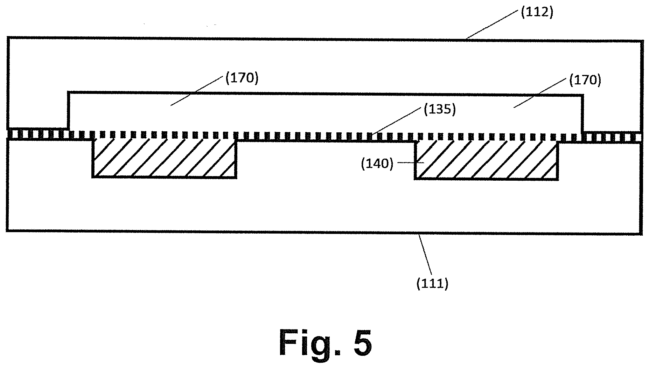

Figur 5 zeigt einen Querschnitt einer erfindungsgemäßen Trägerplatteneinheit (110), bestehend aus einer ersten Trägerplatte (111) und einer zweiten Trägerplatte (112) die über der ersten Trägerplatte (111) angeordnet ist. Zwischen der ersten Trägerplatte (111) und der zweiten Trägerplatte (112) ist eine Trennvorrichtung, insbesondere Membran (135) angeordnet. Diese trennt die Kultivierungskammern (140) der ersten Trägerplatte (111) von den Medienkammern (170) der zweiten Trägerplatte (112).

Figur 6 zeigt den schematischen Aufbau einer ersten Trägerplatte (111) mit Zugangsöffnungen (130), die mit einer oder mehreren Kultivierungskammern (140) über Kanal (150) verbunden sind, sowie Durchgangsöffnungen (125) zur Verbindung der Vorrichtung mit einer externen Drehvorrichtung.

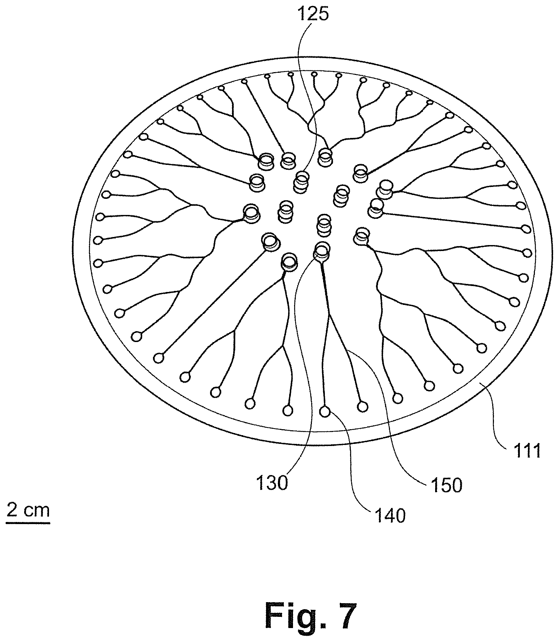

Figur 7 zeigt den Aufbau einer ersten Trägerplatte (111), wobei die Zugangsöffnungen (130), die Kanäle (150) und die Kultivierungskammern (140) zur Hervorhebung mit Tinte eingefärbt sind. Die Durchgangsöffnungen (125) sind nicht eingefärbt. Die Trägerplatte ist mit einer unstrukturierten PDMS-Schicht versiegelt.

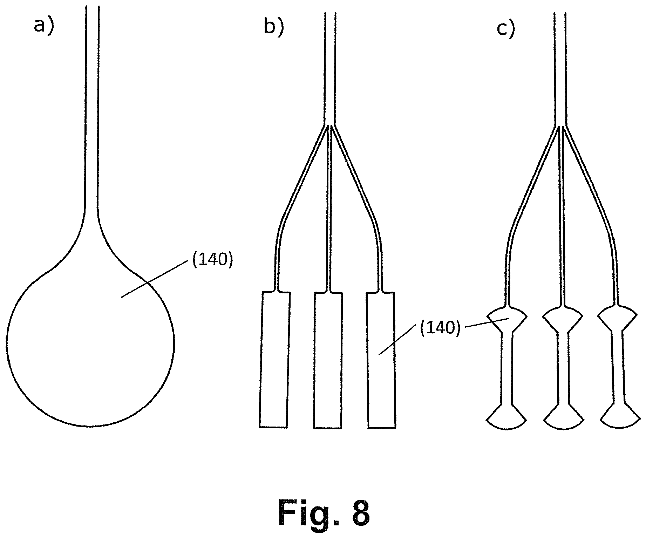

Figur 8 zeigt eine schematische Darstellung möglicher Kultivierungskammergeometrien, a) zeigt eine einzelne runde Kultivierungskammer (140), b) zeigt drei rechteckige Kultivierungskammern (140) und c) zeigt drei hantelförmige Kultivierungskammern (140), die speziell für Kardiomyozyten ausgestaltet sind.

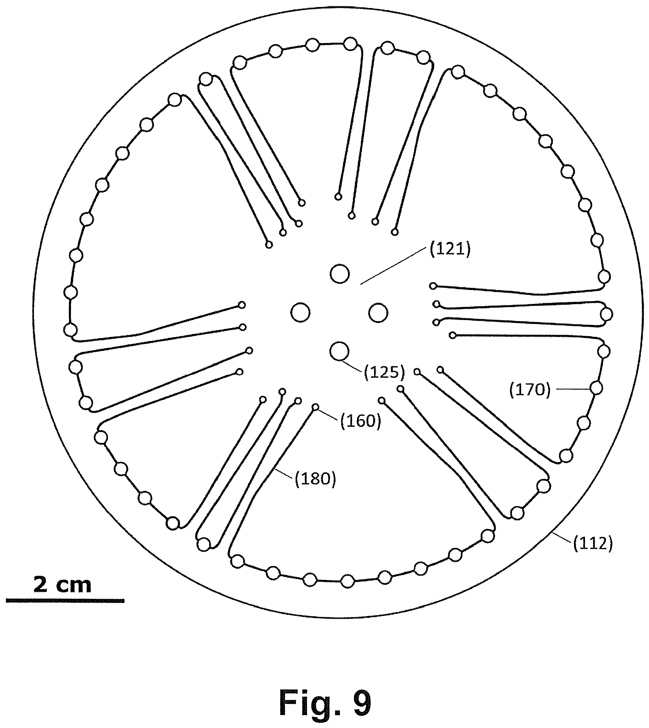

Figur 9 zeigt eine schematische Darstellung der zweiten Trägerplatte (112) mit im Zentralbereich (121) angeordneten Verbindungsvorrichtungen, insbesondere Durchgangsöffnungen (125), Medienöffnungen (160), Medienkammern (170) und die die Medieneinlässe (160) mit den Medienkammern (170) verbindenden Kanäle (180). Hierbei sind jeweils zwei Medienöffnungen (160) mit einer Medienkammer (170) oder mehreren Medienkammern (170) verbunden, die dann in Reihe geschaltet sind.

Figur 10 zeigt den schematischen Aufbau der Trennvorrichtung (135), die zwischen der ersten Trägerplatte (111) und der zweiten Trägerplatte angeordnet ist. Die schwarzen Punkte repräsentieren durchlässige Poren, welche in einem hexagonalen Gitter angeordnet sind. Die unterschiedliche Dichte der Poren stellt Bereiche unterschiedlicher Porosität der Trennvorrichtung (135), insbesondere der Membran dar.

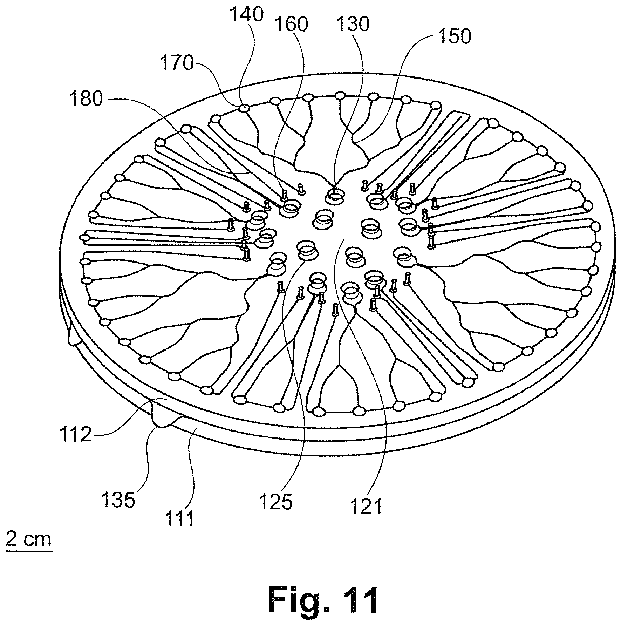

Figur 11 zeigt die erfindungsgemäße Vorrichtung bestehend aus einer ersten Trägerplatte (111), einer Membran (135) und einer zweiten Trägerplatte (112). Im Zentralbereich (121) weist die Vorrichtung vier Verbindungsvorrichtungen, insbesondere Durchgangsöffnungen (125) zur Befestigung der Vorrichtung an einem externen Motor auf. Proximal dazu sind die größeren Zugangsöffnungen (130) und die kleineren Medienöffnungen (160) zu sehen, sowie Kanäle (150) der ersten Trägerplatte (111) und Medienkanäle (180) der zweiten Trägerplatte (112) und die überlappend angeordneten Kultivierungskammern (140) und Medienkammern (170).

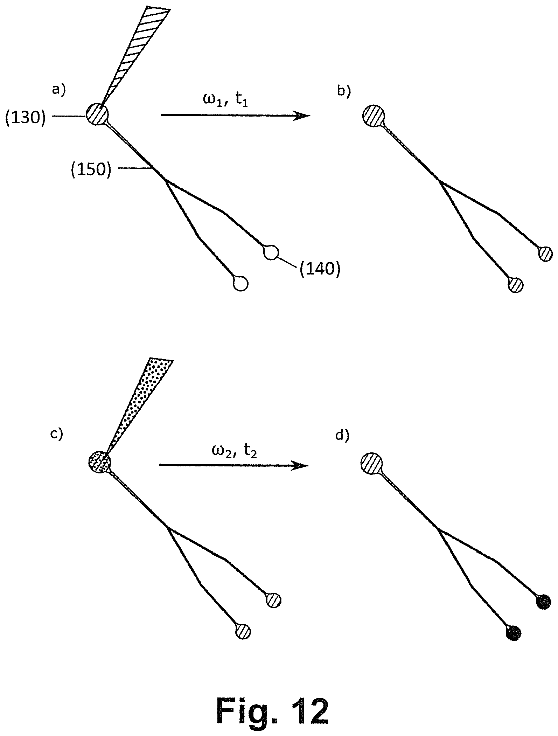

Figur 12 zeigt die zum Beladen der Kultivierungskammern (140) notwendigen Schritte: a) pipettieren von Zellmedium in Zugangsöffnung (130) b) Rotation (ω₁) der Vorrichtung für t₁ sodass alle Kanäle (150) und Kultivierungskammern (140) mit Medium befüllt und Luftblasen entfernt werden, c) pipettieren der Zellsuspension in Zugangsöffnung (130) und d) erneute Rotation (ω₂) für t₂ sodass alle Zellen in die Kultivierungskammern (140) befördert werden und dort bevorzugt in großer Dichte vorliegen.

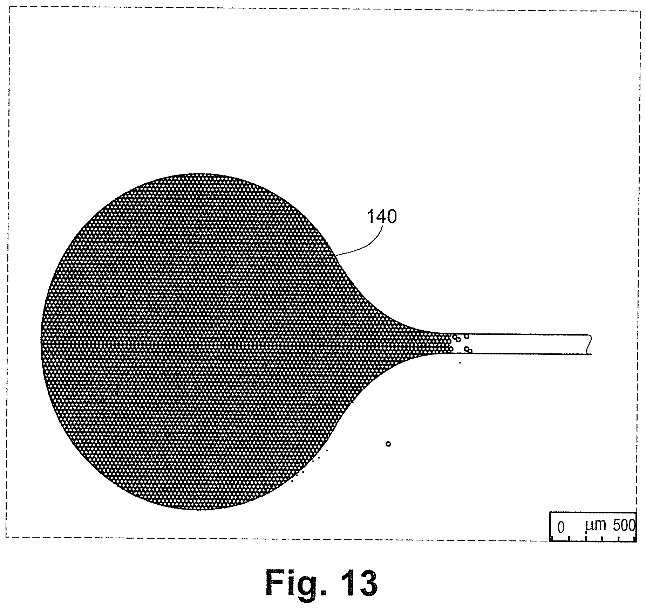

Figur 13 zeigt eine mit Zellen (Fibroblasten) gefüllte runde Kultivierungskammer (140) der ersten Trägerplatte (111). Die Zellen wurden durch Rotation der Trägerplatte in die Kultivierungskammer (140) befördert und dort akkumuliert.

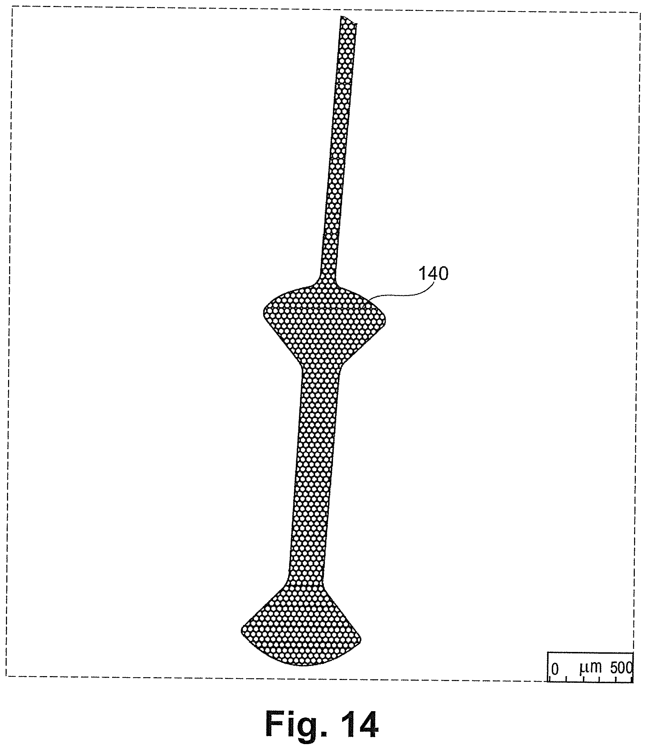

Figur 14 zeigt eine mit Zellen (Kardiomyozyten) gefüllte hantelförmige Kultivierungskammer (140) der ersten Trägerplatte.

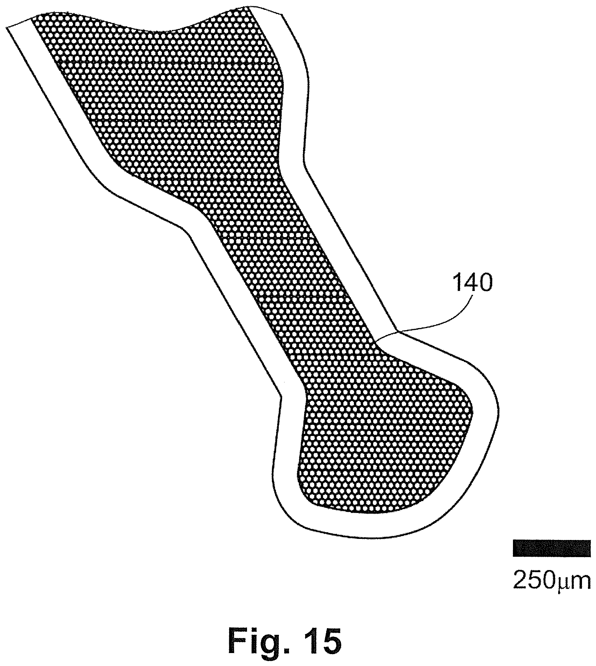

Figur 15 zeigt eine mit Zellen (Fibroblasten) gefüllte lasergeschnittene Kultivierungskammer (140) der ersten Trägerplatte.

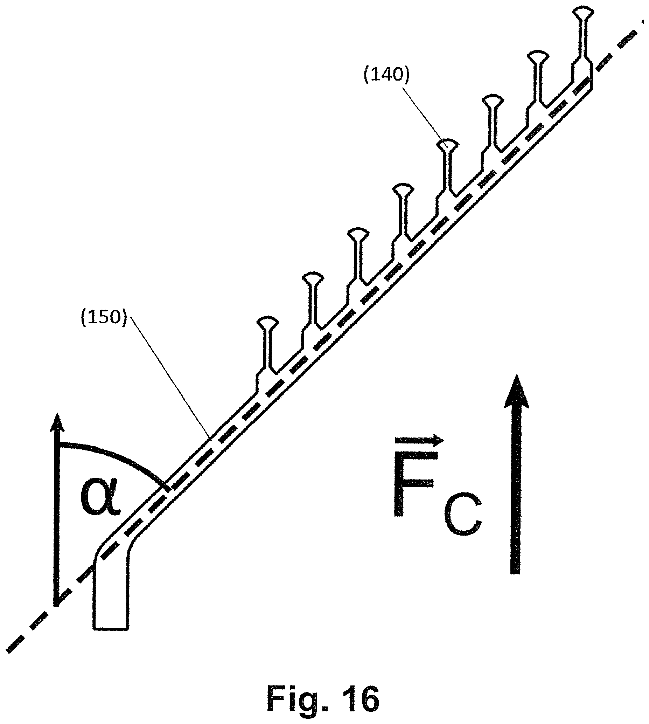

Figur 16 zeigt entlang eines Kanals (150) angeordnete Kultivierungskammern (140). Dabei ist der Kanal (150) um einen definierten Winkel α gegenüber der Richtung der durch Rotation erzeugten Zentrifugalkraft F_{C} geneigt.

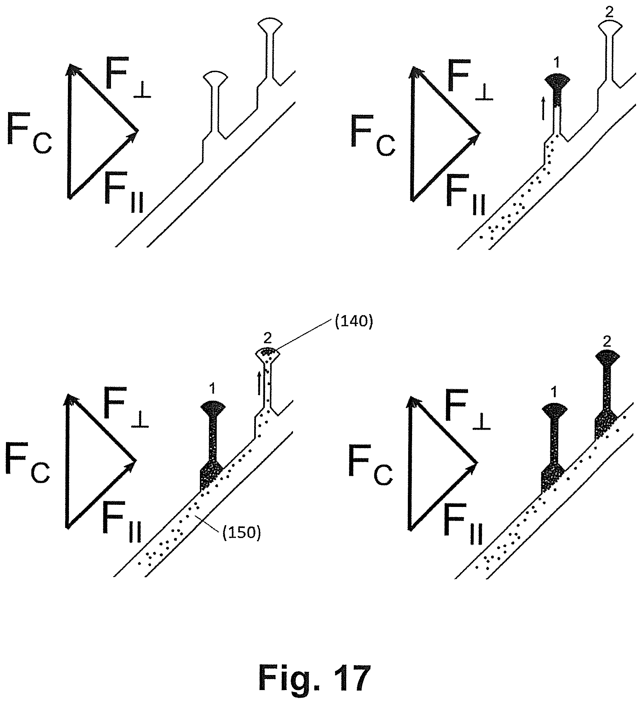

Figur 17 zeigt schematisch die Befüllung der Kultivierungskammern (140) durch die Zentrifugalkraft F_{C}, die sich in die Abtriebskraft F_{II} entlang des um den Winkel α geneigten Kanals (150) und die Anpresskraft F_{⊥}, die senkrecht auf die peripher gelegene Kanalwand des um den Winkel α geneigten Kanal (150) wirkt, aufspalten lässt.

Figur 18 zeigt mittels des erfindungsgemäßen Verfahrens erhaltene dreidimensionale Zellverbände (Kardiomyozyten) in acht Kultivierungskammern (140) der Vorrichtung.

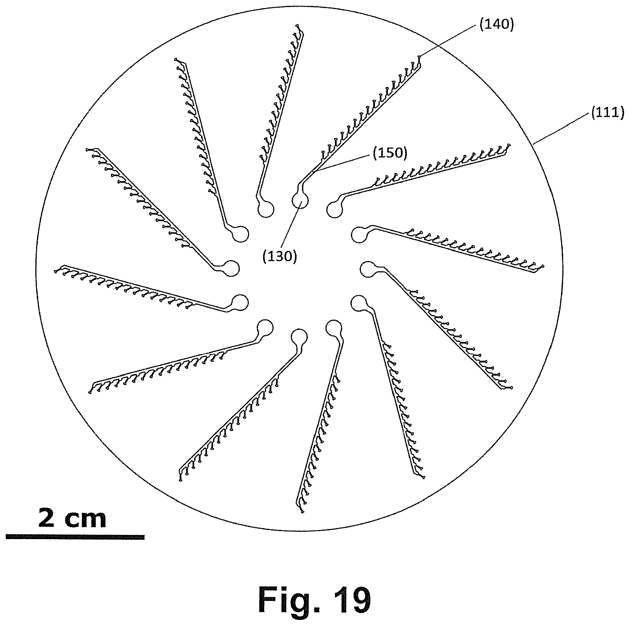

Figur 19 zeigt eine Ausführungsform der erfindungsgemäßen Vorrichtung, bei der auf einer ersten Trägerplatte (111) mehrere um eine zentrale Rotationsachse angeordnete Zugangsöffnungen (130) jeweils über einen geneigten Kanal (150) mit entlang dem Kanal (150) angeordneten Kultivierungskammern (140) verbunden sind.

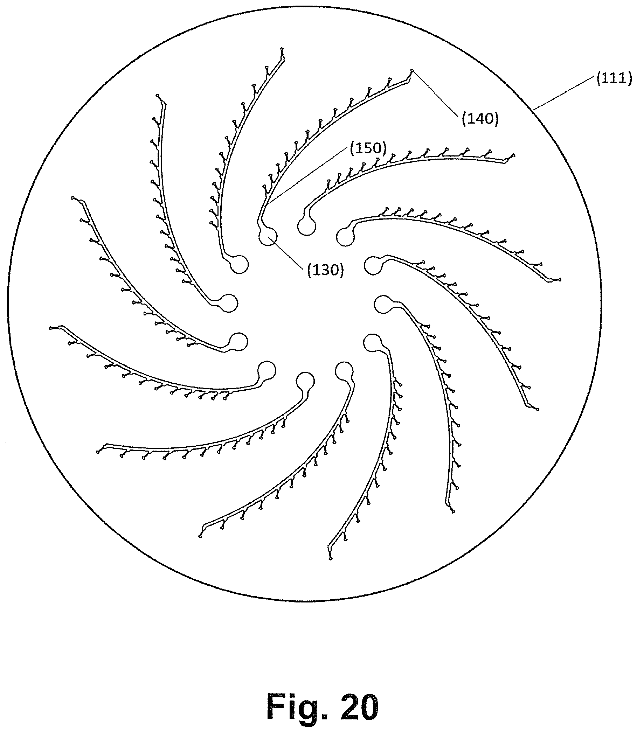

Figur 20 zeigt eine Ausführungsform der erfindungsgemäßen Vorrichtung, bei der auf einer ersten Trägerplatte (111) mehrere um eine zentrale Rotationsachse angeordnete Zugangsöffnungen (130) jeweils über einen gekrümmten Kanal (150) mit entlang dem Kanal (150) angeordneten Kultivierungskammern (140) verbunden sind.

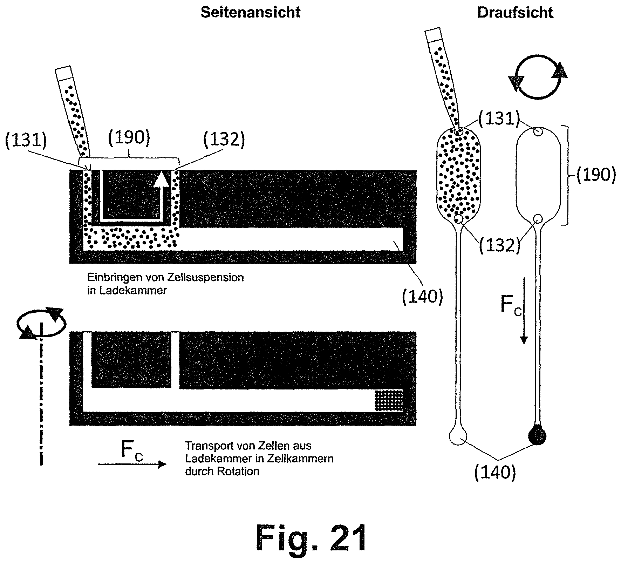

Figur 21 zeigt eine Ausführungsform der erfindungsgemäßen Vorrichtung, bei der die mindestens eine Zugangsöffnung als Ladekammer (190) ausgestaltet ist, welche zwei Zugangsöffnungen (131, 132) umfasst. Durch die Zentrifugalkraft Fc werden die Zellen zur Kultivierungskammern (140) befördert.

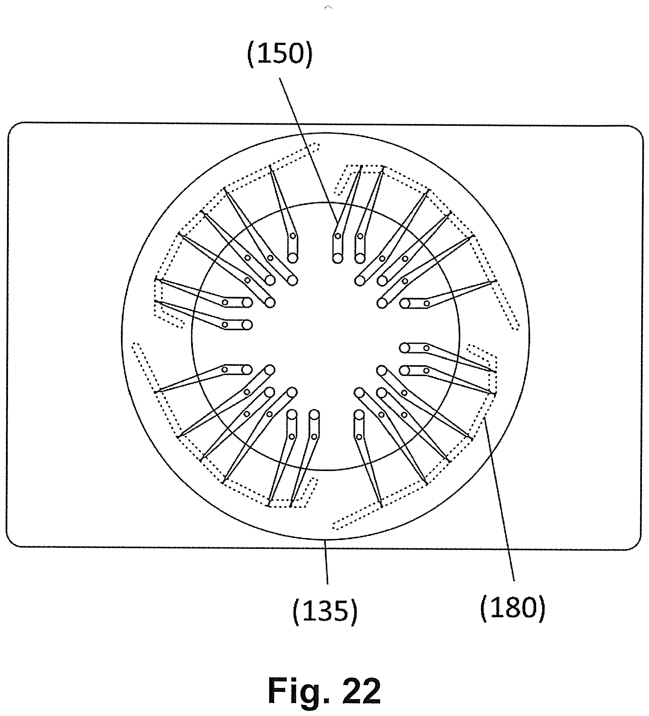

Figur 22 zeigt eine Ausführungsform, bei der die eine zentrale Rotationsachse umfassende Vorrichtung für die Kultivierung von Zellen im Mikrotiterplatten-Format ausgestaltet ist. Gezeigt sind die Kanäle (150), die Medienkanäle (180) und die Membran (135).

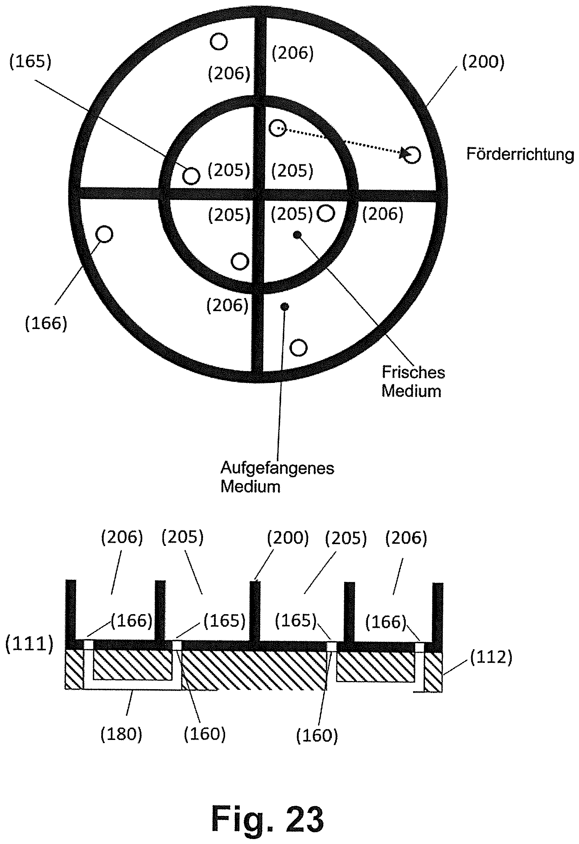

Figur 23 zeigt eine Ausführungsform der erfindungsgemäßen Vorrichtung, bei der die Trägerplatteneinheit (110) zusätzlich ein Reservoir (200) für Flüssigkeiten, insbesondere für Zellkulturmedium oder zu untersuchende Wirkstoffe, umfasst, das über einer zweiten Trägerplatte (112) angeordnet ist und separate proximal zur zentral gelegenen Rotationsachse der Trägerplatteneinheit angeordnete Behältnisse (205) mit Medienauslässen (165) und separate distal zur zentral gelegenen Rotationsachse der Trägerplatteneinheit angeordnete Behältnisse (206) mit Medieneinlässe (166) umfasst, wobei die Medienauslässe (165) jeweils über Medienöffnungen (160) und Medienkanäle (180) der darunterliegenden zweiten Trägerplatte (112) mit den Medieneinlässen (166) in Fluidverbindung stehen.

Figur 24 zeigt den experimentell durch gravimetrische Messung bestimmten Volumenstrom in einer erfindungsgemäßen Vorrichtung in Abhängigkeit von der Rotationsgeschwindigkeit (experimental) im Vergleich zu den rechnerisch bestimmten Werten (theory).

## Patentansprüche

1. Vorrichtung für die Kultivierung von Zellen, umfassend eine eine zentrale Rotationsachse (120) aufweisende Trägerplatteneinheit (110) mit mindestens einer proximal zu der Rotationsachse (120) angeordneten Zugangsöffnung (130), mindestens einer distal zu der Rotationsachse (120) angeordneten Kultivierungskammer (140) und mindestens einem die mindestens eine Zugangsöffnung (130) mit der mindestens einen Kultivierungskammer (140) verbindenden Kanal (150), wobei die Trägerplatteneinheit (110) mindestens eine erste Trägerplatte (111) und eine darüber oder darunter angeordnete zweite Trägerplatte (112) umfasst und wobei die mindestens eine erste Trägerplatte (111) die eine mindestens eine Zugangsöffnung (130), die mindestens eine Kultivierungskammer (140) und den mindestens einen die mindestens eine Zugangsöffnung (130) und die mindestens eine Kultivierungskammer (140) verbindenden Kanal (150) aufweist, und wobei die zweite Trägerplatte (112) mindestens eine Medienöffnung (160), mindestens eine Medienkammer (170) und mindestens einen die mindestens eine Medienöffnung (160) mit der mindestens einen Medienkammer (170) verbindenden Medienkanal (180) aufweist.

2. Vorrichtung nach Anspruch 1, wobei die Trägerplatteneinheit (110) einen Zentralbereich (121) mit mindestens einer Verbindungsvorrichtung, insbesondere mindestens einer Durchgangsöffnung oder mindestens einer Verankerungsvorrichtung (125), für eine Drehvorrichtung aufweist.

3. Vorrichtung nach Anspruch 1 oder 2, wobei die Vorrichtung mindestens eine, vorzugsweise peripher angeordnete, Arretierungsvorrichtung für eine Drehvorrichtung aufweist.

4. Vorrichtung nach einem der vorhergehenden Ansprüche, wobei der mindestens eine Kanal (150) ein verzweigter oder unverzweigter Kanal ist.

5. Vorrichtung nach einem der vorhergehenden Ansprüche, wobei der mindestens eine Kanal (150) die mindestens eine Zugangsöffnung (130) mit mindestens zwei Kultivierungskammer (140) verbindet.

6. Vorrichtung nach einem der vorhergehenden Ansprüche, wobei der Kanal (150) mindestens über einen Teil seiner Länge mindestens zwei unmittelbar an den Kanal (150) angrenzende Kultivierungskammern (140) aufweist.

7. Vorrichtung nach einem der vorhergehenden Ansprüche, wobei der Kanal (150) mindestens über einen Teil seiner Länge gekrümmt ist.

8. Vorrichtung nach Anspruch 7, wobei der Kanal (150) eine statische oder winkelabhängige Krümmung aufweist.

9. Vorrichtung nach einem der vorhergehenden Ansprüche, wobei die mindestens eine Zugangsöffnung (130) als Ladekammer (190) ausgestaltet ist, die mindestens zwei Zugangsöffnungen (131, 132) aufweist.

10. Vorrichtung nach einem der vorhergehenden Ansprüche, wobei der mindestens eine Medienkanal (180) mindestens zwei Medienöffnungen (160) mit mindestens einer, vorzugsweise mindestens zwei Medienkammern (170) verbindet.

11. Vorrichtung nach einem der vorhergehenden Ansprüche, wobei zwischen der ersten Trägerplatte (111) und der zweiten Trägerplatte (112) mindestens eine Trennvorrichtung (135), insbesondere mindestens eine Membran, angeordnet ist.

12. Vorrichtung nach einem der vorhergehenden Ansprüche, wobei die mindestens eine Kultivierungskammer (140) der ersten Trägerplatte (111) und die mindestens eine Medienkammer der zweiten Trägerplatte (112) überlappend ausgestaltet sind und in fluidischer Verbindung stehen.

13. Vorrichtung nach einem der Ansprüche 10 bis 12, wobei die Trägerplatteneinheit (110) zusätzlich ein Reservoir für Flüssigkeiten, insbesondere für Zellkulturmedium oder zu untersuchende Wirkstoffe, umfasst.

14. Vorrichtung nach einem der vorhergehenden Ansprüche, wobei die Trägerplatteneinheit (110) die Form einer Scheibe aufweist.

15. Vorrichtung nach einem der Ansprüche 1 bis 13, wobei die Trägerplatteneinheit (110) als Mikrotiterplatte ausgestaltet ist.

16. Vorrichtung nach einem der vorhergehenden Ansprüche, wobei die Trägerplatteneinheit (110) aus Glas oder einem polymeren Material aufgebaut ist.

17. Vorrichtung nach einem der vorhergehenden Ansprüche, wobei die Trägerplatteneinheit (110) aus Polydimethylsiloxan (PDMS) oder Cycloolefin-Copolymeren (COC) aufgebaut ist.

18. Verfahren zum Kultivieren von Zellen, wobei die Zellen in einer Vorrichtung gemäß einem der Ansprüche 1 bis 17 kultiviert werden.

19. Verfahren nach Anspruch 18, wobei die Kultivierung erfolgt durch:
a) Bereitstellen der Zellen und einer Vorrichtung nach einem der Ansprüche 1 bis 17,
b) Einbringen der Zellen in die Vorrichtung nach einem der Ansprüche 1 bis 17 durch die mindestens eine Zugangsöffnung (130),
c) Einbringen der Vorrichtung nach einem der Ansprüche 1 bis 17 in eine eine Rotation der Vorrichtung ermöglichende Drehvorrichtung,
d) in Rotation versetzen der Vorrichtung nach einem der Ansprüche 1 bis 17,
e) Erhalt von Zellen in der mindestens einen Kultivierungskammer (140) und
f) Kultivieren der Zellen in der mindestens einen Kultivierungskammer (140).

20. Verfahren nach Anspruch 18 oder 19, wobei weiterhin die Verfahrensschritte
g) Einbringen von Zellkulturmedium in die mindestens eine Medienöffnung (160),
h) in Rotation versetzen der Vorrichtung nach einem der Ansprüche 1 bis 17 und
i) Erhalt von Zellkulturmedium in der mindestens einen Medienkammer (170) zur Versorgung der Zellen in der Kultivierungskammer (140)
durchgeführt werden.

21. Verfahren nach einem der Ansprüche 18 bis 20, wobei ein kontinuierliches oder pulsatives Durchströmen der Medienkanäle (180) und Medienkammern (170) mit Zellkulturmedium durch die Erzeugung eines Druckgradienten, externen oder integrierten Pumpen oder durch Rotation der Vorrichtung ermöglicht wird.

22. Verfahren nach Anspruch 18 bis 21, wobei durch das Kultivieren der Zellen in der mindestens einen Kultivierungskammer (140) ein Zellverband, insbesondere ein dreidimensionaler Zellverband, entsteht.

23. Verfahren zur Herstellung eines Zellverbandes, **dadurch gekennzeichnet, dass** ein Verfahren zum Kultivieren von Zellen gemäß einem der Ansprüche 18 bis 22 durchgeführt und ein Zellverband erhalten wird.

24. Verfahren zur Herstellung einer Vorrichtung nach einem der Ansprüche 1 bis 17, wobei in einem ersten Verfahrensschritt mindestens ein die Trägerplatteneinheit bildendes Material, insbesondere polymeres Material, bereitgestellt und dieses in einem Form- und Stabilität gebenden Verfahren, insbesondere einem Photolithographie-, Softlithographie-, selektivem Lasersinter-, Laser-Schneide und Fräs-, Laserablations-, Tintenstrahldruck mit Photopolymeren, Schmelzbeschichtungs-, Laminated Object Manufacturing (LOM-), Stereolithographie-, Heißpräge-, insbesondere Mikroheißprägen, Fräs-, z.B. CNC-Fräsen, insbesondere Mikrofräsen, Kunststoffspritzguss-, insbesondere Mikrospritzguss und/oder 3D-DruckVerfahren, zu einer Vorrichtung gemäß einem der Ansprüche 1 bis 17 ausgestaltet wird.

## Claims

1. A device for cultivating cells, comprising a carrier plate unit (110) which has a central axis of rotation (120) and has at least one access opening (130) arranged proximally to the axis of rotation (120), at least one cultivation chamber (140) arranged distally to the axis of rotation (120), and at least one channel (150) connecting the at least one access opening (130) to the at least one cultivation chamber (140), wherein the carrier plate unit (110) comprises at least one first carrier plate (111) and a second carrier plate (112) arranged above or below it and wherein the first carrier plate (111) has the at least one access opening (130), the at least one cultivation chamber (140), and the at least one channel (150) connecting the at least one access opening (130) and the at least one cultivation chamber (140), and wherein the second carrier plate (112) has at least one media opening (160), at least one media chamber (170), and at least one media channel (180) connecting the at least one media opening (160) to the at least one media chamber (170).

2. The device according to claim 1, wherein the carrier plate unit (110) has a central region (121) having at least one connecting device, in particular at least one through opening or at least one anchoring device (125), for a rotational device.

3. The device according to claim 1 or 2, wherein the device has at least one, preferably peripherally arranged, locking device for a rotational device.

4. The device according to any one of the preceding claims, wherein the at least one channel (150) is a branched or unbranched channel.

5. The device according to any one of the preceding claims, wherein the at least one channel (150) connects the at least one access opening (130) to at least two cultivation chambers (140).

6. The device according to any one of the preceding claims, wherein the channel (150) has at least two cultivation chambers (140) directly adjoining the channel (150) at least over a part of its length.

7. The device according to any one of the preceding claims, wherein the channel (150) is curved at least over a part of its length.

8. The device according to claim 7, wherein the channel (150) has a static or angle-dependent curvature.

9. The device according to any one of the preceding claims, wherein the at least one access opening (130) is designed as a loading chamber (190), which has at least two access openings (131, 132).

10. The device according to any one of the preceding claims, wherein the at least one media channel (180) connects at least two media openings (160) to at least one, preferably at least two media chambers (170).

11. The device according to any one of the preceding claims, wherein at least one separating device (135), in particular at least one membrane, is arranged between the first carrier plate (111) and the second carrier plate (112).

12. The device according to any one of the preceding claims, wherein the at least one cultivation chamber (140) of the first carrier plate (111) and the at least one media chamber of the second carrier plate (112) are formed overlapping and have a fluidic connection.

13. The device according to any one of claims 10 to 12, wherein the carrier plate unit (110) additionally comprises a reservoir for liquids, in particular for cell culture medium or active ingredients to be studied.

14. The device according to any one of the preceding claims, wherein the carrier plate unit (110) has the form of a disk.

15. The device according to any one of claims 1 to 13, wherein the carrier plate unit (110) is designed as a micro-titration plate.

16. The device according to any one of the preceding claims, wherein the carrier plate unit (110) is constructed from glass or a polymer material.

17. The device according to any one of the preceding claims, wherein the carrier plate unit (110) is constructed from polydimethyl siloxane (PDMS) or cycloolefin copolymers (COC).

18. A method for cultivating cells, wherein the cells are cultivated in a device according to any one of claims 1 to 17.

19. The method according to claim 18, wherein the cultivation is performed by:
a) providing the cells and a device according to any one of claims 1 to 17,
b) introducing the cells into the device according to any one of claims 1 to 17 through the at least one access opening (130),
c) introducing the device according to any one of claims 1 to 17 into a rotational device enabling a rotation of the device,
d) setting the device according to any one of claims 1 to 17 into rotation,
e) receiving cells in the at least one cultivation chamber (140), and
f) cultivating the cells in the at least one cultivation chamber (140).

20. The method according to claim 18 or 19, wherein furthermore the method steps
g) introducing cell culture medium into the at least one media opening (160),
h) setting the device according to any one of claims 1 to 17 into rotation, and
i) receiving cell culture in the at least one media chamber (170) to supply the cells in the cultivation chamber (140)
are carried out.

21. The method according to any one of claims 18 to 20, wherein a continuous or pulsed flow through the media channels (180) and media chambers (170) with cell culture medium is enabled by the generation of a pressure gradient, external or integrated pumps, or by rotation of the device.

22. The method according to claims 18 to 21, wherein a cell complex, in particular a three-dimensional cell complex, results due to the cultivation of the cells in the at least one cultivation chamber (140).

23. A method for producing a cell complex, **characterized in that** a method for cultivating cells according to any one of claims 18 to 22 is carried out and a cell complex is obtained.

24. A method for producing a device according to any one of claims 1 to 17, wherein in a first method step, at least one material, in particular polymer material, forming the carrier plate unit is provided and this is formed into a device according to any one of claims 1 to 20 in a method providing shape and stability, in particular a photolithography, soft lithography, selective laser sintering, laser cutting and milling, laser ablation, inkjet printing using photopolymers, melt coating, laminated object manufacturing (LOM), stereolithography, hot embossing, in particular micro-hot embossing, milling, for example CNC milling, in particular micro-milling, plastic injection molding, in particular micro-injection molding, and/or 3D printing method.

## Revendications

1. Dispositif pour la culture de cellules, comprenant un module de substrats (110) présentant un axe de rotation central (120) avec au moins une ouverture d'accès (130) disposée de manière proximale à l'axe de rotation (120), au moins une chambre de culture (140) disposée de manière distale à l'axe de rotation (120) et au moins un canal (150) connectant l'au moins une ouverture d'accès (130) à l'au moins une chambre de culture (140), dans lequel le module de substrats (110) comprend au moins un premier substrat (111) et un second substrat (112) disposé au-dessus ou en dessous de lui, et dans lequel l'au moins un premier substrat (111) présente l'au moins une ouverture d'accès (130), l'au moins une chambre de culture (140) et l'au moins un canal (150) connectant l'au moins une ouverture d'accès (130) et l'au moins une chambre de culture (140), et dans lequel le second substrat (112) présente au moins une ouverture de milieu (160), au moins une chambre de milieu (170) et au moins un canal de milieu (180) connectant l'au moins une ouverture de milieu (160) à l'au moins une chambre de milieu (170).

2. Dispositif selon la revendication 1, dans lequel le module de substrats (110) présente une région centrale (121) avec au moins un dispositif de connexion, notamment au moins une ouverture de passage ou au moins un dispositif d'ancrage (125), pour un dispositif de rotation.

3. Dispositif selon la revendication 1 ou 2, dans lequel le dispositif présente au moins un dispositif de blocage pour un dispositif de rotation, disposé de préférence de manière périphérique.

4. Dispositif selon une des revendications précédentes, dans lequel l'au moins un canal (150) est un canal ramifié ou non ramifié.

5. Dispositif selon une des revendications précédentes, dans lequel l'au moins un canal (150) connecte l'au moins une ouverture d'accès (130) à au moins deux chambres de culture (140).

6. Dispositif selon une des revendications précédentes, dans lequel le canal (150) présente au moins deux chambres de culture (140) directement adjacentes au canal (150) au moins sur une partie de sa longueur.

7. Dispositif selon une des revendications précédentes, dans lequel le canal (150) est courbé au moins sur une partie de sa longueur.

8. Dispositif selon la revendication 7, dans lequel le canal (150) présente une courbure statique ou dépendant de l'angle.

9. Dispositif selon une des revendications précédentes, dans lequel l'au moins une ouverture d'accès (130) est configurée en tant que chambre de chargement (190) qui présente au moins deux ouvertures d'accès (131, 132).

10. Dispositif selon une des revendications précédentes, dans lequel l'au moins un canal de milieu (180) connecte au moins deux ouvertures de milieu (160) à au moins une, de préférence au moins deux chambres de milieu (170).

11. Dispositif selon une des revendications précédentes, dans lequel au moins un dispositif de séparation (135), notamment au moins une membrane, est disposé entre le premier substrat (111) et le second substrat (112).

12. Dispositif selon une des revendications précédentes, dans lequel l'au moins une chambre de culture (140) du premier substrat (111) et l'au moins une chambre de milieu du second substrat (112) sont configurées de manière chevauchante et sont en connexion fluidique.

13. Dispositif selon une des revendications 10 à 12, dans lequel le module de substrats (110) comprend en outre un réservoir pour fluides, notamment pour milieu de culture cellulaire ou substances actives à analyser.

14. Dispositif selon une des revendications précédentes, dans lequel le module de substrats (110) présente la forme d'un disque.

15. Dispositif selon une des revendications 1 à 13, dans lequel le module de substrats (110) est configuré en tant que plaque de microtitration.

16. Dispositif selon une des revendications précédentes, dans lequel le module de substrats (110) est construit en verre ou un matériau polymère.

17. Dispositif selon une des revendications précédentes, dans lequel le module de substrats (110) est construit en polydiméthylsiloxane (PDMS) ou copolymères de cyclooléfine (COC).

18. Procédé de culture de cellules, dans lequel les cellules sont cultivées dans un dispositif selon une des revendications 1 à 17.

19. Procédé selon la revendication 18, dans lequel la culture s'effectue par :
a) mise à disposition des cellules et d'un dispositif selon une des revendications 1 à 17,
b) introduction des cellules dans le dispositif selon une des revendications 1 à 17 à travers l'au moins une ouverture d'accès (130),
c) introduction du dispositif selon une des revendications 1 à 17 dans un dispositif de rotation permettant une rotation du dispositif,
d) mise en rotation du dispositif selon une des revendications 1 à 17,
e) obtention de cellules dans l'au moins une chambre de culture (140) et
f) culture des cellules dans l'au moins une chambre de culture (140).

20. Procédé selon la revendication 18 ou 19, dans lequel les étapes de procédé
g) introduction de milieu de culture cellulaire dans l'au moins une ouverture de milieu (160),
h) mise en rotation du dispositif selon une des revendications 1 à 17 et
i) obtention de milieu de culture cellulaire dans l'au moins une chambre de milieu (170) pour l'alimentation des cellules dans la chambre de culture (140)
sont en outre réalisées.

21. Procédé selon une des revendications 18 à 20, dans lequel un passage continu ou pulsatif des canaux de milieu (180) et chambres de milieu (170) avec du milieu de culture cellulaire est permis par la génération d'un gradient de pression, des pompes externes ou intégrées ou par rotation du dispositif.

22. Procédé selon une des revendications 18 à 21, dans lequel une structure cellulaire, notamment une structure cellulaire tridimensionnelle, apparaît par la culture des cellules dans l'au moins une chambre de culture (140).

23. Procédé de fabrication d'une structure cellulaire, **caractérisé en ce qu'**un procédé de culture de cellules selon une des revendications 18 à 22 est réalisé et une structure cellulaire est obtenue.

24. Procédé de fabrication d'un dispositif selon une des revendications 1 à 17, dans lequel au moins un matériau formant le module de substrats, notamment du matériau polymère, est mis à disposition dans une première étape de procédé et celui-ci est configuré en un dispositif selon une des revendications 1 à 17 dans un procédé donnant forme et stabilité, notamment un procédé de photolithographie, lithographie molle, frittage laser sélectif, coupe laser et fraisage, ablation laser, impression jet d'encre avec des photopolymères, enduction par fusion, fabrication d'objet par laminage (LOM), stéréolithographie, estampage à chaud, notamment micro-estampage à chaud, fraisage, par exemple fraisage CNC, notamment micro-fraisage, moulage par injection de plastique, notamment micro-moulage par injection et/ou impression 3D.
